# EUROPEAN PATENT SPECIFICATION

(11) **EP 3 437 607 B1**
(45) Date of publication and mention of the grant of the patent: **06.11.2019**
(21) Application number: 16907359.0
(22) Date of filing: 01.07.2016
(51) Int. Cl.: A61F 13/511, A61F 13/514, A61F 13/532, A61F 13/533, A61F 13/539, A61F 13/512, A61F 13/513, A61F 13/15

(54) **ABSORBENT ARTICLE**
SAUGFÄHIGER ARTIKEL
ARTICLE ABSORBANT

(30) Priority: 30.06.2016 JP 2016131101
(43) Date of publication of application: 06.02.2019
(73) Proprietor: Unicharm Corporation, Ehime 799-0111 (JP)
(72) Inventor: MIYAMA, Takuya, Kanonji-shi Kagawa 769-1602 (JP); SAKAGUCHI, Satoru, Kanonji-shi Kagawa 769-1602 (JP); UDA, Masashi, Kanonji-shi Kagawa 769-1602 (JP)
(74) Representative: Dolleymores
(86) International application number: PCT/JP2016/069720
(87) International publication number: WO 2018/003126

(56) References cited:
- EP-A1- 2 901 976
- EP-A1- 2 901 989
- WO-A1-2004/022831
- WO-A1-2013/005782
- JP-A- H02 175 958
- JP-A- 2002 306 533
- JP-A- 2003 102 772
- JP-A- 2003 235 896
- JP-A- 2009 148 328
- JP-A- 2014 204 798
- JP-A- 2016 077 900
- JP-B1- 5 829 326

## Description

### FIELD

The present invention relates to an absorbent article, such as a sanitary napkin, disposable diaper or incontinence pad.

### BACKGROUND

Among absorbent articles such as disposable diapers and sanitary napkins, research has been carried out on absorbent articles that employ cotton nonwoven fabrics including cotton as natural fibers, for structural members such as a top sheet, in order to obtain the feeling of assurance that is provided by natural materials.

As an example of such an absorbent article, PTL 1 proposes an absorbent article in which there are provided a top sheet constructed of a cotton nonwoven fabric and an absorbent body as a lower layer below the top sheet, and there is additionally inserted between them a heat-fusible fiber sheet having lower fiber density and hydrophilicity than the cotton nonwoven fabric, with numerous embossings being formed from the front surface side in the layered state. The absorbent article disclosed in PTL 1 is said to allow moisture absorbed into the top sheet to rapidly penetrate into the interior, without impairing flexibility.

### [CITATION LIST]

### [PATENT LITERATURE]

[PTL 1] Japanese Unexamined Patent Publication No. 2009-148328

EP2901976A1 discloses an absorbent article comprising a liquid-permeable top sheet, a liquid-impermeable back sheet and an absorbent body between the top sheet and the back sheet, wherein the top sheet includes a nonwoven fabric having a plurality of projections and a plurality of recesses, the projections and recesses each containing a blood slipping agent with a kinematic viscosity of 0.01 to 80 mm²/s at 40°C, a water holding percentage of 0.01 to 4.0 mass% and a weight-average molecular weight of less than 1,000.

### SUMMARY

### [TECHNICAL PROBLEM]

However, when a cotton nonwoven fabric with high water retention is provided as a top sheet on the upper side of an absorbent body, as in the absorbent article disclosed in PTL 1, and excreted fluid such as urine absorbed into the absorbent body has been released from the absorbent body as moisture by evaporation and the like, it becomes absorbed and held in the cotton nonwoven fabric provided on the upper side of the absorbent body, and as a result the wearer perceives a condition of dampness or feels mustiness, potentially producing a feeling of discomfort.

In addition, when the skin of the wearer is wetted by excreted fluid such as urine or moisture from it, the skin of the wearer becomes damp, and it is difficult to maintain resistance to chemical and physical irritation (i.e., the protective function of the skin against various types of irritation is reduced). Furthermore, when such dampened skin is altered to an alkaline state due to ammonia components from excreted fluid such as urine (i.e., it is subjected to chemical irritation) or undergoes physical irritation by friction against the top sheet, the skin is unable to withstand such irritation and may exhibit eruption (dermatitis).

It is therefore an object of the present invention to provide an absorbent article using a nonwoven fabric that includes cotton as the top sheet, wherein such issues as causing perception of a condition of wetness by the wearer, a musty feel, or discomfort for the wearer are unlikely to occur, and skin eruption is also less likely to occur.

### [SOLUTION TO PROBLEM]

One aspect (aspect 1) of the invention is an absorbent article including a liquid-permeable top sheet situated on the side facing the skin of the wearer, a liquid-impermeable back sheet situated on the side not facing the skin of the wearer, and an absorbent body situated between both sheets, wherein the top sheet is a nonwoven fabric comprising at least two fiber layers including a first fiber layer composed of cotton and thermoplastic resin fibers and a second fiber layer composed of hydrophobic thermoplastic resin fibers, the nonwoven fabric having a first surface which is the skin-facing side surface, formed by the second fiber layer, and a second surface which is the non-skin-facing side surface, facing the absorbent body, the nonwoven fabric being provided with a plurality of protruded sections protruding toward the first surface and a plurality of recesses depressed toward the second surface, and formed between adjacent protruded sections, the protruded sections having spaces faced by the second surface of the nonwoven fabric, and the nonwoven fabric exhibiting weak acidity at least at the protruded sections.

According to the absorbent article of aspect 1, the top sheet is a nonwoven fabric comprising at least two fiber layers, including a first fiber layer composed of cotton and thermoplastic resin fibers, and a second fiber layer composed of hydrophobic thermoplastic resin fibers, the nonwoven fabric being provided with a plurality of protruded sections protruding toward the first surface side and a plurality of recesses depressed toward the second surface side, and the protruded sections having spaces faced by the second surface of the nonwoven fabric, and therefore even when excreted fluid such as urine excreted from a wearer and absorbed into the absorbent body has been released from the absorbent body as moisture by evaporation and the like, the moisture is absorbed and held in the cotton of the first fiber layer while being retained in a state of moisture in the spaces (i.e. the spaces are in a highly humid state), such that a state of gas-liquid equilibrium is formed between the moisture in the spaces (the gas phase) and the excreted fluid absorbed and held in the absorbent body (the liquid phase), and further discharge of moisture from the absorbent body can be inhibited.

Furthermore, since the first surface of the nonwoven fabric, which is the surface facing the skin of the wearer, is formed by the second fiber layer, excreted fluid such as urine that has been absorbed and held in the cotton of the first fiber layer can be prevented from contacting with the skin of the wearer, and the skin of the wearer is also less likely to become damp.

Furthermore, even when excreted fluid such as urine (including moisture released from the absorbent body) has permeated the second fiber layer and contacted with the skin of the wearer, since the nonwoven fabric exhibits weak acidity at least at the protruded sections that tend to contact with the skin of the wearer, the skin of the wearer is unlikely to be altered to alkalinity (i.e., the skin of the wearer is unlikely to be subjected to chemical irritation), and skin eruption (dermatitis) is less likely to occur.

With the absorbent article of aspect 1, therefore, there is low likelihood of creating a perception of a wetted state or causing a musty feel or the like for the wearer, which could produce discomfort for the wearer, and skin eruption is also less likely to be produced.

An absorbent article according to another aspect of the invention (aspect 2) is the absorbent article of aspect 1 in which the back sheet has air permeability.

Since the absorbent article of aspect 2 has air permeability in the liquid-impermeable back sheet, moisture released from the absorbent body to the non-skin-facing side can be discharged through the back sheet, and it is possible to reduce the amount of moisture in the absorbent article or moisture trapped between the absorbent article and the skin of the wearer.

With the absorbent article of aspect 2, therefore, the wearer is less likely to feel mustiness and the skin of the wearer is less likely to become damp by moisture, and as a result, skin eruption is less likely to be produced.

An absorbent article according to yet another aspect of the invention (aspect 3) is an absorbent article according to aspect 1 or 2 including a cotton fiber mass in the first fiber layer.

With the absorbent article of aspect 3, since the first fiber layer situated on the second surface (non-skin-facing surface) side of the nonwoven fabric includes a cotton fiber mass, moisture (excreted fluid) released from the absorbent body is absorbed and held in the cotton fiber mass in the first fiber layer in a concentrated (spot-like) manner, and it is possible to reduce the area of the sections where moisture has been absorbed and held in the in-plane direction of the first fiber layer (in a spot-like form), thereby making it possible to minimize the amount of moisture released from the first surface side of the first fiber layer. As a result, moisture that has been released from the absorbent body can be effectively confined in the spaces of the protruded sections, and the skin of the wearer is even less likely to become damp.

An absorbent article according to yet another aspect of the invention (aspect 4) is the absorbent article according to any one of aspects 1 to 3 wherein the absorbent article has a back surface film on the non-skin-facing side of the back sheet, the back surface film having lower air permeability than the back sheet.

With the absorbent article of aspect 4, which has a back surface film with lower air permeability than the back sheet on the non-skin-facing side of the back sheet, an air permeability gradient is formed in which the air permeability is lower from the back sheet toward the back surface film, and moisture released from the absorbent body toward the non-skin-facing side (i.e., the back sheet side) tends to be drawn into the back surface film through the back sheet, and be eliminated to the exterior of the absorbent article. As a result, the amount of moisture released from the absorbent body to the skin-facing side (i.e., the opposite side from the back sheet) can be reduced, and the wearer is less likely to feel mustiness and the skin of the wearer is less likely to become damp by moisture, and as a result, skin eruption is less likely to be produced.

An absorbent article according to yet another aspect of the invention (aspect 5) is an absorbent article according to any one of aspects 1 to 4, wherein the protruded sections are laid out in a first direction on the first surface of the nonwoven fabric and provided at predetermined intervals in a second direction that is perpendicular to the first direction, the recesses are laid out in the first direction and provided between adjacent protruded sections in the second direction, the recesses each having a first recess comprising a first bottom section situated more toward the second surface side than the location of the first surface side at the top sections of the protruded sections, and a plurality of second recesses inside the first recess, provided in a discontinuous manner in the first direction, and depressed from the first bottom section toward the second surface, wherein each of the second recesses comprises perimeter wall section extending from the first bottom section in the direction of the second surface side, and second bottom section having the highest fiber density of the nonwoven fabric, provided on the edge of the second surface side of the perimeter wall section, so as to plug the edge.

The absorbent article of aspect 5 is constructed so that the fiber density on the second bottom sections of the second recesses located between adjacent protruded sections, in the nonwoven fabric composing the aforementioned top sheet, is the highest in the nonwoven fabric, and in the thickness direction of the nonwoven fabric, it has a density gradient from the protruded sections with relatively low fiber density to the second bottom sections of the second recesses having the highest fiber density, such that excreted fluid such as urine supplied from the first surface of the nonwoven fabric is easily drawn in a spot-like manner from the protruded sections to the second bottom sections of the second recesses, allowing excellent absorption performance (especially absorption rate and liquid migration properties) to be exhibited as a top sheet, and helping to prevent moisture that has been released from the absorbent body, from migrating from the second surface side to the first surface side of the nonwoven fabric. As a result, moisture released from the absorbent body can be effectively confined in the spaces of the protruded sections, and the wearer is even less likely to feel mustiness and the skin of the wearer is even less likely to become damp by moisture, and consequently skin eruption is even less likely to be produced.

When the amount of cotton in the fiber layer is increased, the flexibility of the fiber layer is reduced by the rigidity of the cotton, while the cushioning property and feel on the skin of the layered nonwoven fabric including the fiber layer, as well as its following property for the shape of the body, can sometimes be impaired; however, with the absorbent article of aspect 5, due to the relatively low fiber density of the protruded sections and the first recesses that tend to contact with the skin of the wearer, as well as the fact that the sections that most easily tend to contact with the skin of the wearer are the top sections of the protruded sections that have small contact area and the fact that stress in the thickness direction acting from the first surface side of the nonwoven fabric can be buffered by deformation of the first recesses in the second surface side, it is possible to ensure adequate flexibility even when the top sheet is composed of a nonwoven fabric that includes cotton.

An absorbent article according to yet another aspect of the invention (aspect 6) is the absorbent article of aspect 5 wherein the pitch of the second recesses in the second direction is no greater than 2.0 mm.

With the absorbent article of aspect 6, since the pitch of the second recesses in the second direction is no greater than 2.0 mm, excreted fluid such as urine supplied to the first surface of the nonwoven fabric is easily drawn up in a spot-like manner into the second bottom sections of the second recesses disposed between adjacent protruded sections, such that the effect exhibited by the absorbent article of aspect 5 is exhibited even more prominently. In addition, in the absorbent article of aspect 6, the cotton in the first fiber layer is easily held in the second bottom sections of the second recesses, thereby helping to prevent tearing of the fiber layer at the second bottom sections and allowing the nonwoven fabric (top sheet) to have excellent strength.

An absorbent article according to yet another aspect of the invention (aspect 7) is the absorbent article according to any one of aspects 1 to 6 wherein the protruded sections are laid out in the first direction of the first surface of the nonwoven fabric and are provided at predetermined intervals in a second direction that is perpendicular to the first direction, while the protruded sections are laid out up to at least one of the edges in the first direction of the absorbent body, or exceeding at least one of the edges.

According to the absorbent article of aspect 7, it is possible for not only moisture released from the skin-facing side of the absorbent body but also moisture released from the edges (side surfaces) in the first direction of the absorbent body to be confined in the spaces of the protruded sections, and therefore the wearer is even less likely to feel mustiness and the skin of the wearer is even less likely to become damp by moisture, and as a result, skin eruption is even less likely to be produced.

An absorbent article according to yet another aspect of the invention (aspect 8) is the absorbent article according to any one of aspects 1 to 7 wherein the absorbent body has a compressed section on the non-skin-facing side surface.

Since the absorbent article of aspect 8 has a compressed section on the non-skin-facing side surface of the absorbent body and excreted fluid absorbed into the absorbent body tends to pool in the compressed section while being less likely to remain on the skin-facing side surface of the absorbent body, it is possible to reduce the amount of moisture released from the absorbent body to the skin-facing side, and the wearer is even less likely to feel mustiness and the skin of the wearer is even less likely to become damp by moisture, and as a result, skin eruption is even less likely to be produced.

An absorbent article according to yet another aspect of the invention (aspect 9) is the absorbent article according to aspect 8 wherein the absorbent article has a lengthwise direction, a widthwise direction and a thickness direction that are mutually perpendicular, the compressed section is provided on the non-skin-facing side surface of the absorbent body, as a linear compressed section running in the lengthwise direction and/or the widthwise direction, and the absorbent body has a plurality of mutually separated punctiform stamped sections on the skin-facing side surface.

Since the absorbent article of aspect 9 has a linear compressed section on the non-skin-facing side surface of the absorbent body, running in the lengthwise direction and/or the widthwise direction, and excreted fluid absorbed into the absorbent body easily pools in a state diffused along the linear compressed section running in the lengthwise direction and/or the widthwise direction, while little thereof remains on the skin-facing side surface of the absorbent body, it is possible to reduce the amount of moisture released from the absorbent body to the skin-facing side, and to make it even less likely that the wearer will feel mustiness.

Furthermore, since it has a plurality of punctiform compressed sections on the skin-facing side surface of the absorbent body, i.e. it has punctiform compressed sections that have high fiber density and function as liquid-absorbing sections utilizing capillary movement, excreted fluid such as urine is easily absorbed in a spot-like manner into the absorbent body and is less likely to be released through the punctiform compressed sections to the skin-facing side, and therefore the amount of moisture released from the absorbent body to the skin-facing side can be reduced, and the wearer is even less likely to experience a feeling of mustiness.

In addition, the skin of the wearer is even less likely to become damp by moisture, and as a result, skin eruption is even less likely to occur.

An absorbent article according to yet another aspect of the invention (aspect 10) is the absorbent article according to any one of aspects 1 to 9 wherein the absorbent article has a lengthwise direction, a widthwise direction and a thickness direction that are mutually perpendicular, the top sheet having a center region running in the lengthwise direction and including the lengthwise axial line of the absorbent article, as viewed flat, and a pair of outside regions located in both side sections adjacent to the center region in the widthwise direction and running in the lengthwise direction, the absorbent article having joining sections at the sections of the second surface sides of the recesses of the nonwoven fabric, that join with the absorbent body, the joining sections extending in the lengthwise direction between the nonwoven fabric and the absorbent body and being disposed in a plurality of rows in the widthwise direction, the joining sections being disposed so that, among the intervals between adjacent joining sections in the widthwise direction, the intervals between joining sections lying in the outside regions of the top sheet are larger than the intervals between joining sections lying in the center region in the widthwise direction of the top sheet.

In the absorbent article of aspect 10, the pitch of the joining sections lying in the center region of the top sheet to which excreted fluid such as urine has been supplied are disposed more densely than the joining sections in the outside regions, and it is therefore possible to firmly join the top sheet and the absorbent body at the center region that greatly contributes to absorption of excreted fluids, while also allowing the spaces of the protruded sections at the center region to be formed more compactly and in greater number than in the outside regions, thereby helping to reliably ensure spaces to hold moisture released from the absorbent body to the skin-facing side.

### [ADVANTAGEOUS EFFECTS OF INVENTION]

According to the present invention it is possible to provide an absorbent article using a nonwoven fabric including cotton as the top sheet, wherein such issues as causing perception of a condition of wetness by the wearer, a musty feel, or discomfort for the wearer are unlikely to occur, and skin eruption is also less likely to occur.

### BRIEF DESCRIPTION OF DRAWINGS

FIG. 1 is a plan view of a disposable diaper according to an embodiment of the invention.
FIG. 2 is a magnified cross-sectional view of the disposable diaper according to an embodiment of the invention shown in FIG. 1, along line II-II'.
FIG. 3 is a perspective view of a nonwoven fabric (fiber laminate) composing the top sheet of a disposable diaper according to an embodiment of the invention.
FIG. 4 is a partial enlarged plan view of a top sheet of a disposable diaper according to the embodiment of the invention.
FIG. 5 is a magnified cross-sectional view of the disposable diaper according to the embodiment of the invention shown in FIG. 4, along line V-V'.
FIG. 6 is a magnified cross-sectional view of the disposable diaper according to the embodiment of the invention shown in FIG. 4, along line VI-VI'.
FIG. 7 is a magnified cross-sectional view of the disposable diaper according to the embodiment of the invention shown in FIG. 4, along line VII-VII'.

### DESCRIPTION OF EMBODIMENTS

Preferred embodiments of the absorbent article of the invention will now be described in detail with reference to the accompanying drawings. Throughout the present description, unless otherwise specified, the simple phrase "as viewed flat" will be used to mean that the absorbent article in the deployed flat state is viewed from the top sheet side in the thickness direction. Also throughout the present description, the term "skin-facing side" means the side that is relatively near the skin of the wearer in the thickness direction of the absorbent article, when the wearer of the absorbent article has worn the absorbent article, and "non-skin-facing side" means the side that is relatively far from the skin of the wearer in the thickness direction of the absorbent article, when the wearer of the absorbent article has worn the absorbent article.

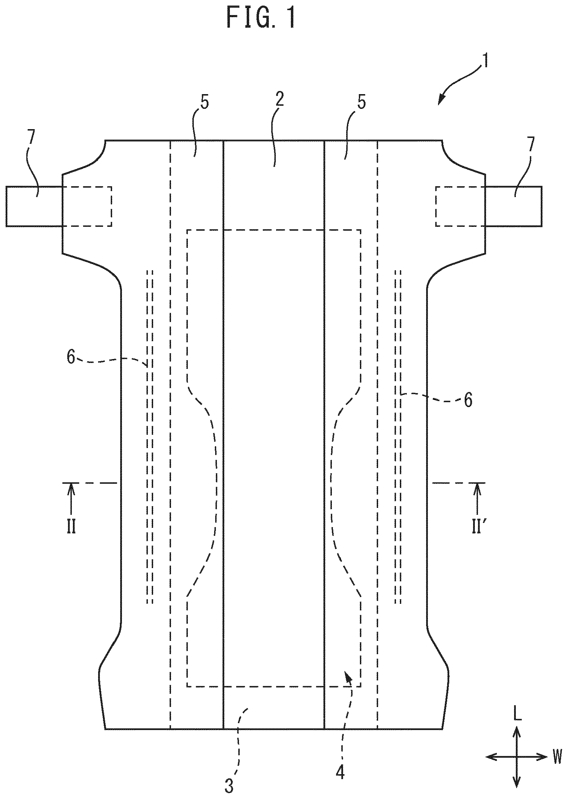

Fig. 1, Fig. 2 and Fig. 4 to Fig. 7 are diagrams schematically showing a disposable diaper 1 (absorbent article) according to an embodiment of the invention. The disposable diaper 1 has a lengthwise direction L, a widthwise direction W and a thickness direction T that are mutually perpendicular, and comprises, as the basic construction, a liquid-permeable top sheet 2 situated on the side facing the skin of the wearer and having the specific protrusion-recess structure described below, a liquid-impermeable back sheet 3 situated on the side not facing the skin of the wearer, and an absorbent body 4 situated between the two sheets.

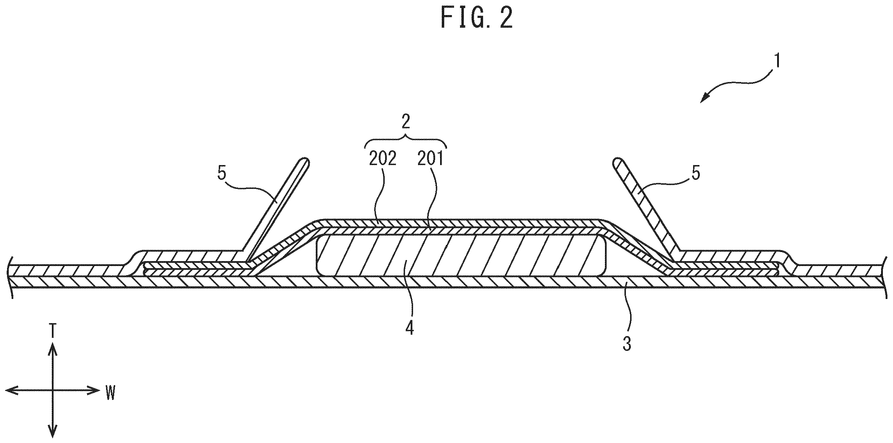

As shown in Fig. 1 and Fig. 2, the disposable diaper 1 further comprises a pair of anti-leakage wall sections 5, 5 made of a pair of side sheets, an elastic member 6 made of rubber or the like for stretching of the section around the left and right legs in contact with the femoral region of the wearer, in the lengthwise direction L, and connecting tape 7 for connection between the abdominal region and dorsal region of the disposable diaper when it is worn, with an outer sheet (not shown) being provided on the non-skin-facing side of the back sheet 3.

The back sheet 3 of this embodiment is provided on the disposable diaper 1 on the side not facing the skin of the wearer (the lower side of the absorbent body 4 in Fig. 2), serving to prevent permeation of excreted fluid that has been discharged by the wearer and to prevent leakage of the excreted fluid into underwear or clothing. The back sheet is not particularly restricted so long as it has properties allowing it to be used as a back sheet for an absorbent article (for example, liquid impermeability, air permeability and flexibility), and for example, a publicly known liquid-impermeable sheet member such as an air-permeable resin film such as polyethylene or polypropylene, or a laminate of a nonwoven fabric attached to such a resin film, may be used. Among these, the back sheet is preferably impermeable to fluids such as excreted fluids but has a prescribed degree of air permeability. If the back sheet has such air permeability, moisture released from the absorbent body to the non-skin-facing side can be released through the back sheet, and it is possible to reduce the amount of moisture in the absorbent article or moisture trapped between the absorbent article and the skin of the wearer.

Incidentally, the back sheet 3 of this embodiment, while sandwiching the absorbent body 4 against the top sheet 2, is mutually joined with the top sheet 2 at the perimeter sections.

The absorbent body 4 of this embodiment absorbs and retains excreted fluid such as urine that has permeated the top sheet 2, and it comprises an absorbent material that can absorb and retain excreted fluid. As shown in Fig. 1, the absorbent body 4 is formed long in the direction along the lengthwise direction L of the disposable diaper 1, in the planar view, with an approximately hourglass-shaped outer form that is concave toward the inner side in the widthwise direction at approximately the center section in the lengthwise direction L, and with a smaller area than the top sheet 2 and back sheet 3. The absorbent body 4 is formed approximately flat on both the surface of the top sheet side (i.e., the skin-facing side) and the surface of the back sheet side (i.e., the side not facing the skin of the wearer), and it has an essentially constant thickness across the entire absorbent body 4. According to the invention, the structure of the absorbent body is not limited to the aspect described above, and the absorbent body may have any outer shape such as rectangular or oblong, for example, and it may also have a thickness that differs in parts.

According to the invention, the absorbent body is not particularly restricted so long as the effect of the invention is not inhibited, and any absorbent body known in the relevant field may be used. Examples of such absorbent bodies include absorbent materials containing either or both water-absorbent fibers such as pulp and a superabsorbent polymer, that are covered by at least one liquid-permeable cover sheet such as a tissue.

Furthermore, the thickness, basis weight and other properties of the absorbent body are not particularly restricted, and may be adjusted as appropriate for the properties to be provided for an absorbent body in an absorbent article such as a disposable diaper (including absorption properties, strength and lightweight properties). For example, the thickness of the absorbent body will usually be in the range of 0.1 to 15 mm, and is preferably in the range of 1 to 10 mm and more preferably in the range of 2 to 5 mm, while the basis weight of the absorbent body will usually be in the range of 20 to 1000 g/m², and is preferably in the range of 50 to 800 g/m² and more preferably in the range of 100 to 500 g/m². Incidentally, according to the invention the thickness and basis weight of the absorbent body may be constant across the entire absorbent body, or they may partially differ.

The absorbent body 4 of this embodiment has the top sheet 2 on the surface of the top sheet side (the upper side of the absorbent body 4 in Fig. 2) and the back sheet 3 on the surface of the back sheet side (the lower side of the absorbent body 4 in Fig. 2) joined with an adhesive such as a hot-melt adhesive.

The top sheet 2 of this embodiment is disposed on the skin-facing side in the thickness direction T of the disposable diaper 1 (specifically, it is disposed on the skin-facing side of the absorbent body 4), and is constructed of a liquid-permeable sheet member that can directly contact with the side of the skin of the wearer. The top sheet 2 can rapidly absorb or transmit excreted fluid such as urine discharged from the wearer, allowing it to migrate toward the absorbent body 4. Incidentally, as shown in Fig. 1, the top sheet 2 is formed long across the entire region in the direction along the lengthwise direction L of the disposable diaper 1.

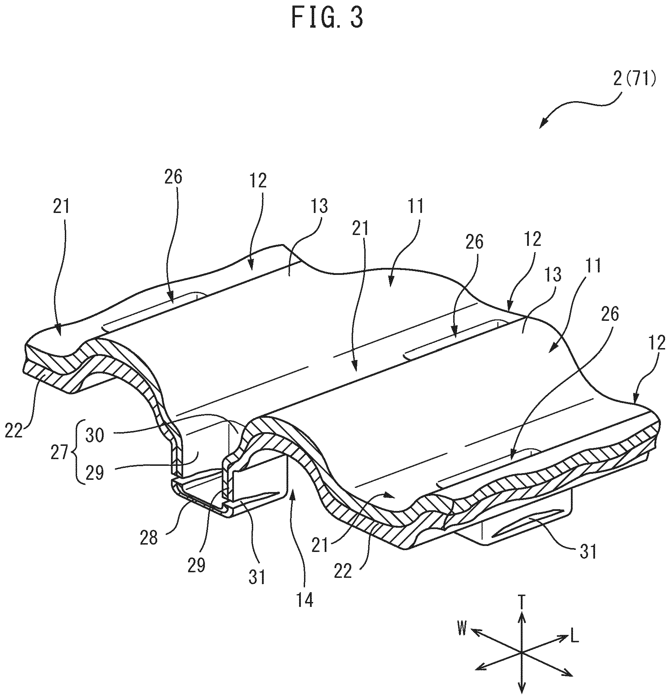

For this embodiment, from the viewpoint of improving the feeling of assurance and absorption properties provided by natural materials, the top sheet 2 used is a nonwoven fabric comprising a two-layer fiber laminate 200, including a first fiber layer 201 that is composed of cotton 203 and thermoplastic resin fibers 204 and that forms the second surface 2b (lower side) of the top sheet 2, and a second fiber layer 202 that is composed of hydrophobic thermoplastic resin fibers 205, as a fiber layer adjacent to the surface of the upper side of the first fiber layer 201 and forming a first surface 2a (upper side) of the top sheet 2. As shown in Fig. 3, the nonwoven fabric has a protrusion-recess structure comprising a plurality of protruded sections 11 protruding toward the direction of the first surface 2a and a plurality of recesses 12 provided between the adjacent protruded sections 11 and depressed toward the direction of the second surface 2b, the protruded sections 11 having spaces 14 faced by the second surface 2b of the nonwoven fabric.

As explained below, the nonwoven fabric is constructed so as to exhibit weak acidity at least at the protruded sections 11 that tend to contact with the skin of the wearer (for this embodiment, incidentally, the thermoplastic resin fibers 205 composing the second fiber layer 202 are covered with a weak acidifying agent as described below, so as to exhibit weak acidity over the entirety of the first surface 2a of the nonwoven fabric, including the protruded sections 11 and the recesses 12).

If the top sheet 2 is composed of a nonwoven fabric having such a protrusion-recess structure, then even when excreted fluid such as urine from a wearer absorbed into the absorbent body 4 has been released from the absorbent body 4 as moisture by evaporation and the like, the moisture can be absorbed and held in the cotton 203 of the first fiber layer 201 while being retained in a state of moisture in the spaces 14 (i.e. the spaces 14 are in a highly humid state, allowing the moisture to be retained), such that a state of gas-liquid equilibrium is formed between the moisture in the spaces 14 (the gas phase) and the excreted fluid absorbed and held in the absorbent body 4 (the liquid phase), and further discharge of moisture from the absorbent body 4 can be inhibited.

Furthermore, since the first surface 2a of the nonwoven fabric forming the top sheet 2, which is the surface facing the skin of the wearer, is formed by the second fiber layer 202, excreted fluid such as urine that has been absorbed and held in the cotton 203 of the first fiber layer 201 can be prevented from contact with the side of the skin of the wearer, and the skin of the wearer is also less likely to become damp. In addition, even when excreted fluid such as urine (including moisture released from the absorbent body) has permeated the second fiber layer 202 and contacted with the skin of the wearer, since the nonwoven fabric exhibits weak acidity at least at the protruded sections 11 that tend to contact with the skin of the wearer, the skin of the wearer is unlikely to be altered to alkalinity, and skin eruption (dermatitis) is less likely to occur.

With the disposable diaper 1 of this embodiment, therefore, which is provided with such a specific top sheet 2, there is low likelihood of creating the perception of a wetted state or causing a musty feel or the like for the wearer, which could produce discomfort for the wearer, and skin eruption is also less likely to be produced.

As used herein, "exhibits weak acidity" means being "weakly acidic" (pH = 3.0 to 6.0) when the front surface of the nonwoven fabric is evaluated by the weak acidity evaluation method described below, or when the pH of the front surface of the nonwoven fabric is measured using a commercially available pH meter.

### <Weak acidity evaluation method>

(1) A bromothymol blue solution (BTB solution) is diluted with a neutral buffering solution to prepare a pH indicator for evaluation of weak acidity.
(2) The pH indicator is either dropped onto the front surface of the nonwoven fabric to be measured, using a single drop dropper, or it is sprayed with an atomizer.
(3) The front surface of the nonwoven fabric on which the pH indicator has adhered is lightly pressed with a glass rod to allow the pH indicator to penetrate the fibers composing the nonwoven fabric.
(4) The color of the front surface of the nonwoven fabric is visually confirmed 10 seconds after the pH indicator has been adhered, and the color is compared with previously prepared color samples for different pH values, to assess the pH of the front surface of the nonwoven fabric. A nonwoven fabric front surface exhibiting a pH of 3.0 to 6.0 is judged as "weakly acidic".

The color samples for the different pH values are prepared in the following manner.
(i) Several types of solutions of known pH (for example, several types with pH values differing by 1.0 each or 0.5 each) (for example, pH buffer solutions of different types with different pH values, or dilutions of a pH buffer solution) are prepared as color sample standard solutions.
(ii) The pH indicator is dropped in a suitable amount onto each of the prepared color sample standard solutions for coloring, to prepare color samples for each pH.

The first fiber layer of the nonwoven fabric (fiber laminate) that is to form the top sheet for the absorbent article of the invention will now be described.

According to the invention, the first fiber layer of the nonwoven fabric that is to form the top sheet is composed essentially of cotton and thermoplastic resin fibers. The cotton in the first fiber layer is not particularly restricted so long as it does not inhibit the effect of the invention, and for example, cotton having a fineness in the range of 1.0 to 15 dtex and a fiber length in the range of 5 to 40 mm may be used. Cotton having a fiber length of 20 mm or greater is preferably used since, when the first fiber layer is laminated with the second fiber layer described below (i.e., when the fiber laminate is formed), it will be possible for a portion of the cotton fibers to readily infiltrate to the interior of the second fiber layer, and to increase the liquid permeability of the nonwoven fabric. Also, blended cotton which is a blend of cotton having a fiber length of 20 mm or greater and cotton having a fiber length of 10 mm or smaller is particularly preferred for use because the liquid permeability of the nonwoven fabric can be increased by the cotton with a fiber length of 20 mm or greater, while the bulk of the nonwoven fabric can be increased by the cotton with a fiber length of 10 mm or smaller.

The content of cotton in the first fiber layer is not particularly restricted so long as it does not inhibit the effect of the invention; however, from the viewpoint of water absorption, water retention, flexibility and the like, it is, for example, in the range of 1 to 70 mass% and preferably in the range of 2 to 30 mass%. Moreover, the cotton in the first fiber layer preferably contains the cotton fiber mass in the first fiber layer, and most preferably the cotton fiber mass is dispersed in a matrix composed of aggregates of the thermoplastic resin fibers. If such a cotton fiber mass is included in the first fiber layer situated on the second surface side (non-skin-facing side) of the top sheet, then when excreted fluid such as urine that has been absorbed and held in the absorbent body has been released from the absorbent body as moisture, the moisture can be absorbed and held in a concentrated (spot-like) manner in the cotton fiber mass in the first fiber layer, and the area of the sections where moisture has been absorbed and held in the in-plane direction of the first fiber layer can be reduced (to a spot-like form), therefore making it possible to further minimize the amount of moisture released from the first surface side of the first fiber layer. As a result, moisture that has been released from the absorbent body can be effectively confined in the spaces of the protruded sections of the nonwoven fabric, as described below.

Furthermore, the thermoplastic resin fibers in the first fiber layer are not particularly restricted so long as they are fibers made of a thermoplastic resin, and examples for the thermoplastic resin include publicly known resins including olefin-based resins such as polyethylene (PE), polypropylene (PP) and ethylene-vinyl acetate copolymer (EVA), polyester-based resins such as polyethylene terephthalate (PET) and polylactic acid (PLA), and polyamide-based resins such as 6-nylon, any of which resins may be used alone or as combinations of two or more of such resins.

Also, the structure of the fibers made of such thermoplastic resins is not particularly restricted, and examples include composite fibers such as core-sheath fibers, side-by-side fibers and island/sea fibers; hollow type fibers; irregular cross-sectional fibers such as flat, Y-shaped or C-shaped fibers; solid crimped fibers with latent crimping or developed crimping; and splittable fibers that are split by a physical load such as a water stream, heat or embossing, and such fibers may be used alone, or two or more different types may be used in combination.

The fineness of the thermoplastic resin fibers is not particularly restricted; however, from the viewpoint of nonwoven fabric strength and flexibility, feel on the skin and liquid permeability, it will usually be in the range of 1.1 to 8.8 dtex and is preferably in the range of 1.5 to 4.6 dtex. Also, the fineness of the thermoplastic resin fibers in the first fiber layer is preferably smaller than the fiber size (fineness) of the hydrophobic thermoplastic resin fibers in the second fiber layer, described below. If the thermoplastic resin fibers in the first fiber layer have a smaller fiber size (fineness) than the hydrophobic thermoplastic resin fibers in the second fiber layer, then the thermoplastic resin fibers with a small fiber size in the first fiber layer will readily become entangled with the cotton that is also in the first fiber layer and with the thermoplastic resin fibers in the second fiber layer, and problems such as tearing of the fiber layer or interlayer separation between the fiber layers, that occur due to dissociation between the cotton and thermoplastic resin fibers, will be less likely to occur, thus allowing the nonwoven fabric to maintain excellent strength even when the nonwoven fabric includes cotton as a constituent component.

The fiber lengths of the thermoplastic resin fibers in the first fiber layer are not particularly restricted; however, from the viewpoint of nonwoven fabric strength, flexibility and liquid permeability, they are usually in the range of 20 to 100 mm and are preferably in the range of 35 to 65 mm. Incidentally, the thermoplastic resin fibers may be subjected to hydrophilicizing treatment, such hydrophilicizing treatment being, for example, treatment utilizing a surfactant, hydrophilic agent or the like (for example, kneading of a surfactant into the fiber interiors, or coating of the fiber surfaces with a surfactant).

The content of thermoplastic resin fibers in the first fiber layer is not particularly restricted; however, from the viewpoint of nonwoven fabric strength, flexibility and the like, it is, for example, in the range of 30 to 99 mass% and preferably in the range of 70 to 98 mass%.

As mentioned above, the first fiber layer is essentially composed of cotton and thermoplastic resin fibers; however, it may also include fibers other than the cotton and thermoplastic resin fibers, or optional additives, within a range such that the effect of the invention is not inhibited.

The second fiber layer of the nonwoven fabric (fiber laminate) that is to form the top sheet for the absorbent article of the invention will now be described.

According to the invention, the second fiber layer of the nonwoven fabric that is to form the top sheet is composed essentially of hydrophobic thermoplastic resin fibers. Such hydrophobic thermoplastic resin fibers are not particularly restricted so long as they have hydrophobicity, and any desired thermoplastic resin fibers may be used. If the second fiber layer forming the skin-facing surface (first surface) of the top sheet is composed of hydrophobic thermoplastic resin fibers, then the second fiber layer will be less likely to absorb and hold excreted fluid and or its moisture, and therefore the perception of a condition of wetness, or production of discomfort, for the wearer will be less likely to occur.

The term "hydrophobic" as used herein means a property of being poorly compatible with water or resistant to holding moisture, and for example, it corresponds to a contact angle of about 80° to 100° with ion-exchanged water. The contact angle with ion-exchanged water may be measured by the method described in "Measurement of initial contact angle" in Japanese Unexamined Patent Publication No. 2005-324010.

Moreover, when the fiber laminate is composed of two fiber layers consisting of a first fiber layer and a second fiber layer it is preferred, for the hydrophobic thermoplastic resin fibers composing the second fiber layer, to use the same type of fibers as the thermoplastic resin fibers in the first fiber layer (i.e., fibers of the same material as the thermoplastic resin fibers in the first fiber layer, and similar fiber lengths), from the viewpoint of easier entanglement between the fibers.

Incidentally, as mentioned above, the second fiber layer is essentially composed of hydrophobic thermoplastic resin fibers; however, it may also include fibers other than the thermoplastic resin fibers mentioned above, or optional additives, within a range such that the effect of the invention is not inhibited.

Also according to the invention, the second fiber layer of the nonwoven fabric forming the top sheet is constructed so as to exhibit weak acidity at least at the protruded sections that tend to contact with the skin of the wearer. The means for constructing the second fiber layer so as to exhibit weak acidity is not particularly restricted and may be a method of adhering a weak acidifying agent onto the second fiber layer, for example, and more specifically, it may be a method in which fibers for forming the second fiber layer (i.e., hydrophobic thermoplastic resin fibers) are covered with a weak acidifying agent by dipping and then the fibers are used to form the second fiber layer, or a method in which the second fiber layer is formed first, after which the surface of the skin-facing side of the second fiber layer (i.e., the surface that includes at least the protruded sections) is coated with a weak acidifying agent.

According to the invention, the weak acidifying agent that may be used in the second fiber layer is not particularly restricted so long as it can render the surface of the skin-facing side of the second fiber layer (i.e., the first surface) weakly acidic, and examples include organic acids such as fatty acids, and oils such as plant-derived fats or oils. Organic acids include fatty acids, such as saturated fatty acids such as succinic acid and lactic acid; and unsaturated fatty acids such as fumaric acid, oleic acid and undecylenic acid, and oils include plant-derived fats or oils composed mainly of saturated fatty acids, such as coconut oil, palm kernel oil and palm oil; and plant-derived fats or oils composed mainly of unsaturated fatty acids, such as castor oil, tall oil and olive oil. These fatty acids may also include appropriate amounts of alkali metal salts such as sodium salts or potassium salts of the fatty acids.

Among these, from the standpoint of resistance to oxidative decomposition and of more readily exhibiting activity at the temperature of excreted fluid that is discharged by the wearer, unsaturated fatty acids or oils with melting points of no higher than 40°C are preferred, and unsaturated fatty acids or oils with melting points of no higher than 30°C are more preferred.

Incidentally, the aforementioned organic acids and oils may be used alone or in combinations of two or more, and they may also optionally contain additives (for example, antioxidants, emollients, coloring agents or aromatics).

Examples of weak acidifying agents other than the organic acids and oils mentioned above include the water permeability imparting agents disclosed in Japanese Unexamined Patent Publication No. 2014-109078 and Japanese Unexamined Patent Publication No. 2014-231666. These water permeability imparting agents can impart durable water permeability to nonwoven fabrics and are therefore preferred for use.

Moreover, the location where the weak acidifying agent is adhered onto the nonwoven fabric (specifically, the second fiber layer) is not particularly restricted so long as it is a location such that weak acidity is exhibited at least at the protruded sections, and it may be adhered only on the protruded sections, or adhered on both the protruded sections and recesses, or adhered over the entire surface of the nonwoven fabric including the protruded sections and the recesses. Since the protruded sections have a shorter contact time with excreted fluid such as urine compared to the recesses, when the weak acidifying agent is adhered onto the protruded sections, the weak acidifying agent is unlikely to flow with the excreted fluid and flow out from the top sheet at least at the protruded sections, and therefore the function and effect of the weak acidifying agent (i.e., the function and effect whereby the skin of the wearer is made resistant to becoming alkaline (or in other words, the skin of the wearer is more likely to be kept weakly acidic), thus making skin eruption less likely to occur) can be exhibited in a steady manner for prolonged periods. This function and effect is particularly advantageous when excreted fluid such as urine is repeatedly supplied.

When a weak acidifying agent is to be coated onto the front surface of the skin-facing side of the nonwoven fabric, the method of coating is not particularly restricted, and for example, any coating apparatus may be employed, such as an extrusion device comprising a discharge nozzle; or a coating applicator such as a non-contact coater, such as a spiral coater, curtain coater, spray coater or dip coater; or a contact coater.

According to the invention, the nonwoven fabric forming the top sheet is not limited to a two-layer fiber laminate as in the embodiment described above, so long as the effect of the invention can be exhibited, and it may be a fiber laminate comprising three or more fiber layers including the first fiber layer and the second fiber layer. If the first surface of the top sheet contacting with the skin of the wearer of the absorbent article is formed by a second fiber layer containing no cotton or hydrophilic fibers, as according to the invention, excreted fluid such as urine that has been absorbed and held in the cotton of the first fiber layer will be less likely to contact with the skin of the wearer, and it will therefore be less prone to cause the perception of a condition of wetness for the wearer, or to produce discomfort for the wearer, and the skin of the wearer will be less likely to become damp (resulting in a lower likelihood that skin eruption will occur). In addition, if the first surface of the top sheet is formed of a fiber layer containing no cotton, this can prevent impairment of the feel on the skin or reduction in flexibility due to the cotton, and can therefore help maintain an excellent feel on the skin and flexibility as a top sheet.

Moreover, when the nonwoven fabric forming the top sheet is a fiber laminate composed of 3 or more fiber layers, the other fiber layers in addition to the first fiber layer and second fiber layer are not particularly restricted, and for example, they may be fiber layers made of natural fibers such as wool, regenerated fibers such as rayon, or synthetic resin fibers such as thermoplastic resin fibers. Other fiber layers may also include optional additives, within a range such that the effect of the invention is not inhibited.

An aspect in which the nonwoven fabric forming the top sheet is constructed of a fiber laminate composed of 3 or more fiber layers will be described in detail below as another embodiment of the invention.

In the nonwoven fabric forming the top sheet, the basis weight of each fiber layer is not particularly restricted; however, from the viewpoint of strength, flexibility and absorption properties of the nonwoven fabric, each is preferably in the range of, for example, 1 to 60 g/m², and preferably in the range of 10 to 30 g/m². There are also no particular restrictions on the basis weight of the nonwoven fabric as a whole; however, it will usually be in the range of 10 to 100 g/m², preferably in the range of 15 to 75 g/m² and more preferably in the range of 20 to 50 g/m². The thickness of the nonwoven fabric is also not particularly restricted; however, will usually be in the range of 0.1 to 5 mm, preferably in the range of 0.5 to 3 mm and more preferably in the range of 0.8 to 2 mm.

According to the invention, the means for producing the nonwoven fabric before shaping of the protrusion-recess structure (i.e., the fiber laminate) is not particularly restricted, and for example, it may be a method of using fibers for formation of each of the aforementioned fiber layers (i.e., cotton and thermoplastic resin fibers for formation of the first fiber layer and thermoplastic resin fibers for formation of the second fiber layer (including those covered by a weak acidifying agent)) to form a web (fleece) corresponding to each fiber layer, and physically or chemically bonding together the fibers in each web or between the webs.

Specifically, the following procedure may be followed to produce a two-layer fiber laminate including the aforementioned first fiber layer and second fiber layer.
(1) A production apparatus is prepared comprising a conveying apparatus for conveying of a sheet member in one direction, a first stage carding apparatus situated above the conveying apparatus and upstream in the machine direction, a second stage carding apparatus situated above the conveying apparatus and downstream in the machine direction (i.e., downstream from the first stage carding apparatus), and an air-through system heating apparatus situated downstream from the second stage carding apparatus.
(2) Thermoplastic resin fibers for formation of the second fiber layer are supplied to the first stage carding apparatus, and the thermoplastic resin fibers are opened by the pin of a revolving roll in the carding apparatus, to form a web corresponding to the second fiber layer on the conveying surface of the conveying apparatus.
(3) Cotton and thermoplastic resin fibers for formation of the first fiber layer are supplied to the second stage carding apparatus while the web corresponding to the second fiber layer formed on the conveying surface of the conveying apparatus is being conveyed downstream in the machine direction, and the fibers are opened by the pin of a revolving roll in the carding apparatus to form a web corresponding to the first fiber layer, on the web corresponding to the second fiber layer during its conveyance.
(4) The layered web obtained by layering the web corresponding to the first fiber layer on the web corresponding to the second fiber layer is conveyed to the air-through system heating apparatus, and in the heating apparatus, the fibers within the web and between the webs are entangled optionally heat-fusing together the thermoplastic resin fibers while fusing and anchoring the cotton on the front surfaces of the thermoplastic resin fibers, to produce a two-layer fiber laminate comprising the layered first fiber layer and second fiber layer.

When a fiber laminate comprising three or more fiber layers is to be produced, a production apparatus having three or more stages of carding apparatuses is provided and production is carried out by the same procedure described above.

The method of forming the web corresponding to each fiber layer is not limited to the method described above, and a wet method, for example, may be used. The method of bonding the webs is also not restricted to the method described above, and a hydroentangling method or needle punching method, for example, may be employed.

The fiber laminate produced in this manner is provided with the specific protrusion-recess structure described below by any desired protrusion-recess shaping and processing method (for example, the protrusion-recess shaping method disclosed in Japanese Patent Publication No. 5829349 or Japanese Patent Publication No. 5829326), to obtain a nonwoven fabric with a protrusion-recess structure that can be used as the top sheet for the absorbent article of the invention.

The protrusion-recess structure of the top sheet will now be described in further detail, using the disposable diaper 1 of the embodiment described above.

In the disposable diaper 1 of this embodiment, as shown in Figs. 3 to 7, the top sheet 2 has a first surface 2a located on the opposite side from the absorbent body 4 and a second surface 2b on the absorbent body side as the side opposite the first surface 2a, and the top sheet 2 has a protrusion-recess structure comprising a plurality of protruded sections 11 protruding toward the first surface 2a, that are formed in a manner laid out in the lengthwise direction L and arranged at a predetermined interval in the widthwise direction W, and a plurality of recesses 12 depressed toward the second surface 2b, that are laid out in the lengthwise direction L between the adjacent protruded sections 11. The top sheet 2 also has, on the second surface sides of the protruded sections 11, spaces 14 faced by the second surface 2b of the top sheet 2 (nonwoven fabric).

Furthermore, as shown in Figs. 3 to 7, the recesses 12 have first recesses 21 comprising first bottom sections 22 located further toward the absorbent body 4 side than the location of the first surface 2a at the top sections 13 of the protruded sections 11, and a plurality of second recesses 26 provided in a discontinuous manner in the lengthwise direction L inside the first recesses 21, and formed as a depressions opening into the first bottom sections 22. Each of the second recesses 26 comprises perimeter wall sections 27 extending from the first bottom section 22 to the absorbent body side (i.e., in the direction of the second surface side), and a second bottom section 28 having the highest fiber density of the top sheet 2, provided at the edges of the perimeter wall sections 27 on the absorbent body 4 side, so as to plug the edges.

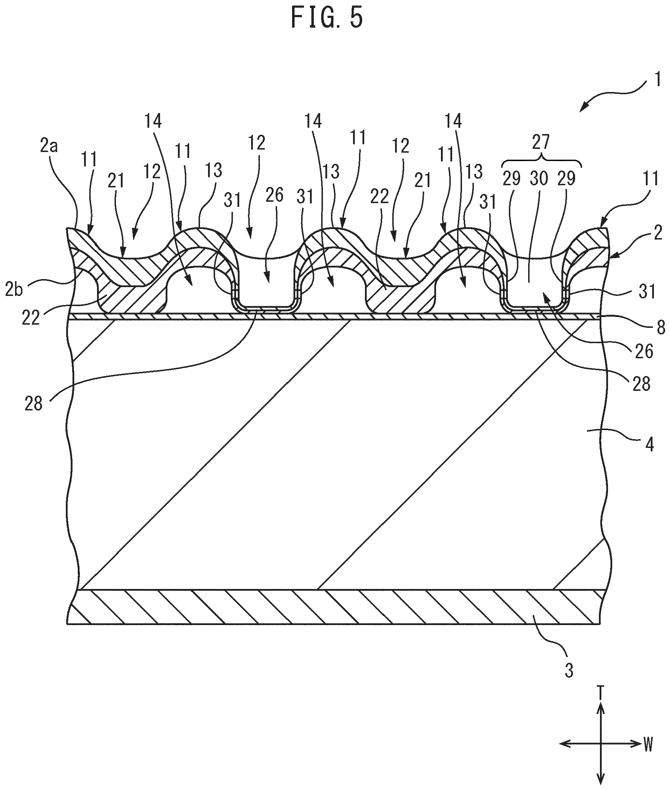

Furthermore, in the top sheet 2 of this embodiment, the second surface 2b is not joined to the absorbent body 4 at the top sections 13 of the protruded sections 11; however, the second bottom sections 28 of the second recesses 26 of the recesses 12 are joined to the sections of the top sheet side of the absorbent body 4 (the surface of the top sheet side of the absorbent body 4, according to this embodiment) by an adhesive layer 8. Also for this embodiment, portions of the second surfaces 2b of the first bottom sections 22 of the first recesses 21 are similarly joined to portions of the top sheet side of the absorbent body 4 by an adhesive layer 8, as shown in Figs. 5 to 7. At the protruded sections 11, on the other hand, portions of the second surfaces 2b including the top sections 13 are not joined to the absorbent body 4.

As shown in Fig. 3, Fig. 5 and Fig. 6, the top sheet 2 of this embodiment has a construction in which the regions of the first surface 2a and the regions of the second surface 2b forming the protruded sections 11 are curved into convex shapes in the direction from the second surface 2b toward the first surface 2a (i.e. in the direction toward the side opposite the absorbent body 4 side). On the other hand, the first recesses 21 of the recesses 12 have a construction in which the regions of the first surface 2a and the regions of the second surface 2b forming the first recesses 21 are curved into convex shapes in the direction from the first surface 2a side toward the second surface 2b side (i.e. the direction toward the absorbent body 4). The top sheet 2 therefore has an essentially wavy cross-sectional shape with alternately repeating protrusions and recesses in the widthwise direction W.

As mentioned above, the protruded sections 11 are laid out in the lengthwise direction L of the first surface 2a (i.e., the lengthwise direction of the top sheet 2), while being disposed in a plurality of rows at predetermined intervals in the widthwise direction W. For this embodiment, each of the protruded sections 11 is laid out continuously in the lengthwise direction L, so as to be mutually parallel with the other protruded sections 11.

Also, although the protruded sections 11 are laid out even beyond both edges in the lengthwise direction (first direction) of the absorbent body for this embodiment, the present invention has no limitation to this aspect, and the protruded sections may be laid out either up to at least one of the edges or beyond one of the edges, in the first direction of the absorbent body. By having the protruded sections laid out in this manner, not only moisture released from the skin-facing side of the absorbent body, but also moisture released from the edges (side surfaces) in the lengthwise direction (first direction) of the absorbent body can be confined to the spaces on the second surface sides of the protruded sections, and can thus make the wearer even less likely to experience a feeling of mustiness.

According to the invention, the intervals between the adjacent protruded sections are not particularly restricted, but are preferably in the range of 0.25 to 5 mm, more preferably in the range of 0.5 to 3 mm and even more preferably in the range of 0.75 to 2 mm. Here, the intervals between adjacent protruded sections are the distances between approximately the center locations of the respective protruded sections in the widthwise direction of the top sheet (essentially the top sections of the protruded sections). If the distance of the intervals between adjacent protruded sections is 0.25 mm or greater, the protrusion-recess structure of the nonwoven fabric will not be too narrow and it will be possible to adequately reduce the contact area between the protruded sections of the nonwoven fabric and the skin of the wearer, so that the feel on the skin of the front surface (first surface) of the nonwoven fabric will be satisfactory. On the other hand, if the distance of the intervals between adjacent protruded sections is 5 mm or smaller, it will be easier to obtain a flexible feel on the skin due to the protrusion-recess structure.

Furthermore, according to the invention, the heights of the protruded sections (i.e., the distances from the first surfaces of the first bottom sections of the first recesses to the top sections of the protruded sections) are not particularly restricted, but are preferably in the range of 0.25 to 5 mm, more preferably in the range of 0.5 to 3 mm and even more preferably in the range of 0.75 to 2 mm. If the heights of the protruded sections are 0.25 mm or greater, protrusion of the protruded sections will not be too small, so that a flexible feel on the skin can be easily obtained due to the protrusion-recess structure. If the heights of the protruded sections are 5 mm or smaller, on the other hand, the protrusions of the protruded sections will not be too large, helping to avoid creation of a sharp structure and thus making it easier to obtain a flexible feel on the skin.

Moreover, in the embodiment described above, the distances between the second surfaces 2b at the top sections 13 of the protruded sections 11, and the top sheet side surface of the absorbent body 4 are smaller than the distances between the sections of the first surfaces 2a of the first bottom sections 22 nearest the absorbent body 4, and the top sheet side surface of the absorbent body 4, as shown in Fig. 5. In other words, the top sections 13 of the protruded sections 11 are disposed, overall, at locations more distant from the top sheet 2 (the upper side in Fig. 5) than the first surfaces 2a of the first bottom sections 22. Because of this, spaces can be stably formed as spaces 14 between the second surfaces 2b of the protruded sections 11 and the top sheet side surface of the absorbent body 4, and when the protruded sections 11 of the top sheet 2 are subjected to external force from the wearer, and particularly shear force in the widthwise direction accompanying friction with the skin of the wearer, the protruded sections 11 deform or collapse, depending on the direction and size of the external force, allowing the external force to be easily buffered. As a result, moisture released from the absorbent body 4 can be more stably controlled, while tensile force of the top sheet 2 generated by friction between the top sheet 2 and the skin of the wearer can be reduced, thus more reliably minimizing detachment between the top sheet 2 and the absorbent body 4.

Incidentally, according to the embodiment described above, the distances between the second surfaces 2b at the top sections 13 of the protruded sections 11 and the surface on the top sheet side of the absorbent body 4 are smaller than the distances between the sections of the first surfaces 2a of the first bottom sections 22 nearest the absorbent body 4 and surface of the top sheet side of the absorbent body 4; however, according to the invention, the relationship between the distances between the second surfaces at the top sections of the protruded sections and the surface of the top sheet side of the absorbent body, and the distances between the sections of the first surfaces of the first bottom sections nearest the absorbent body and the surface of the top sheet side of the absorbent body, does not necessarily need to satisfy such a relationship, so long as detachment between the top sheet and the absorbent body can be stably inhibited.

For this embodiment, the first recesses 21 are formed integrally with the protruded sections 11 in the widthwise direction. Also, the first bottom sections 22 of the first recesses 21 have the greatest maximum thickness among the thickness of the top sheet 2, and the first bottom sections 22 are sections with excellent elasticity as a whole. The plurality of first recesses 21 formed in the top sheet 2 are all formed to be mutually parallel and with equal widths. For this embodiment, the second surfaces 2b of the first bottom sections 22 of the first recesses 21 are joined to the top sheet side surface of the absorbent body 4, at least at the sections of the first bottom sections 22 located nearest the top sheet side, and their nearby sections.

As shown in Fig. 3, the second recesses 26 have essentially rectangular openings in the planar view, and protrude overall to the absorbent body 4 side of the top sheet 2, defining essentially cuboid interior spaces. The second recesses 26 are disposed at a constant interval in the lengthwise direction of each of the recesses 12 (i.e., the lengthwise direction of the first recesses 21), each of the second recesses 26 being formed in a mutually independent manner from the other second recesses 26.

The perimeter wall sections 27 extending from the first bottom sections 22 of the second recesses 26 toward the absorbent body side comprise a pair of first perimeter wall sections 29, 29 formed along the lengthwise direction of the top sheet 2 and a pair of second perimeter wall sections 30, 30 formed along the widthwise direction of the top sheet 2, the pair of first perimeter wall sections 29, 29 being mutually disposed facing each other, and the pair of second perimeter wall sections 30, 30 also being mutually disposed facing each other.

Furthermore, as shown in Fig. 3, Fig. 5 and Fig. 6, each of the pair of first perimeter wall sections 29, 29 has a hole 31 formed running from the interior space of the second recess 26 to the second surface 2b. According to this embodiment, one hole 31 is provided for each of the pair of first perimeter wall sections 29, 29 (i.e., two holes 31 are provided for each second recess 26), and the holes 31 are formed at locations near the second bottom sections 28 of the first perimeter wall sections 29 in the thickness direction T. On the other hand, the pair of second perimeter wall sections 30, 30 do not have corresponding holes 31, each of the second perimeter wall sections 30 having its entire edge, which is located on the absorbent body 4 side, directly connected to the second bottom section 28.

Although providing the top sheet with the second recesses is not an essential constituent feature for the invention, the top sheet preferably has a specific protrusion-recess structure comprising such second recesses. The top sheet has such a specific protrusion-recess structure in order to reduce to a minimum the opportunity for the bottoms of the recesses (more specifically the first bottom sections of the first recesses) to contact the skin of the wearer, and in order to absolutely minimize the contact area even when the first bottom sections have contacted with the skin of the wearer. That is, the top sheet of the invention having the specific protrusion-recess structure contacts most easily with the skin of the wearer in the order of the protruded sections first, followed by the first bottom sections of the first recesses (i.e., the protruded sections that are not joined to the absorbent body and have the highest flexibility in the top sheet contact more easily with the skin of the wearer than the first bottom sections), and since the second recesses are also formed (i.e., sections are formed where the first recesses are not present), considering that a smaller contact area contact with the skin of the wearer is more likely to provide a feeling of flexibility, this further reduces the portions of the first bottom sections of the first recesses contacting with the skin of the wearer and tends to provide the wearer with a more flexible feel. Incidentally, since the second recesses are formed in the first bottom sections of the first recesses, there is less opportunity for contact with the skin of the wearer, and an uncomfortable feeling or sensation of foreign matter due to contact with the second recesses is less likely to be produced.

Furthermore, since the top sheet having such a specific protrusion-recess structure can buffer stress in the thickness direction applied from the first surface side of the top sheet, by deformation of the first recesses toward the second surface side, it is possible to ensure adequate flexibility even when the nonwoven fabric forming the top sheet is one that contains cotton.

Also, the depths of the second recesses (i.e., the distances from the first surfaces (upper side surfaces) of the first bottom sections of the first recesses to the first surfaces of the second bottom sections of the second recesses) are not particularly restricted so long as the effect of the invention is not inhibited; however, they are preferably in the range of 0.05 to 2 mm, more preferably in the range of 0.075 to 1.5 mm and even more preferably in the range of 0.1 to 1 mm. If the depths of the second recesses are 0.05 mm or greater, it will be easier to ensure the rigidity of the second bottom sections as described below, and the strength of the nonwoven fabric in the thickness direction can be adequately ensured. If the depths of the second recesses are 2 mm or smaller, conversely, it will be easier to ensure strength in thickness direction when the other structural members (such as the absorbent body, nonwoven fabric or film) of the absorbent article are attached, and the wearer will be less likely to experience a rigid feel under compression.

The relationship between the depths of the second recesses and the heights of the protruded sections may be such that the depths of the second recesses are preferably in the range of 10 to 80%, more preferably in the range of 15 to 70% and even more preferably in the range of 20 to 60% of the heights of the protruded sections. If the depths of the second recesses are at least 10% of the heights of the protruded sections, then it will be possible to sufficiently ensure the spaces for forming the holes at the perimeter wall sections of the second recesses, thus helping prevent the holes from being formed with inadequate shapes or from failing to be formed, and allowing the aforementioned excellent flexibility to be easily obtained. Conversely, if the depths of the second recesses are no greater than 80% of the heights of the protruded sections, the holes will be unlikely to be formed with excessively large sizes, helping to prevent the strength of the perimeter wall sections of the second recesses from being reduced (which tends to cause napping), and helping to ensure a satisfactory feel of the nonwoven fabric on the skin.

According to the invention, the lengths of the second recesses in the first direction (lengthwise direction) are not particularly restricted, but are preferably in the range of 0.25 to 5 mm, more preferably in the range of 0.5 to 3 mm and even more preferably in the range of 0.75 to 2 mm. If the lengths of the second recesses in the first direction are 0.25 mm or longer, consistent sizes can be ensured for the second recesses, and therefore the aforementioned function and effect exhibited by the second recesses can be adequately exhibited. If the lengths of the second recesses in the first direction are no greater than 5 mm, on the other hand, the second recesses will not be too long in the first direction, and it will be easier to obtain a more flexible feel on the skin compared to a flat nonwoven fabric or a nonwoven fabric without recesses.

The lengths of the second recesses in the second direction (widthwise direction), on the other hand, are not particularly restricted but are preferably in the range of 0.25 to 5 mm, more preferably in the range of 0.5 to 3 mm and even more preferably in the range of 0.75 to 2 mm. If the lengths of the second recesses in the second direction are 0.25 mm or longer, consistent sizes can be ensured for the second recesses, and therefore the aforementioned function and effect exhibited by the second recesses can be adequately exhibited. If the lengths of the second recesses in the second direction are no greater than 5 mm, on the other hand, the second recesses will not be too large, and an uncomfortable feeling or sensation of foreign matter will be less likely to occur when the skin of the wearer has contacted with the nonwoven fabric.

Moreover, the pitch of the second recesses in the second direction (i.e., the distance between adjacent second recesses in the second direction) is not particularly restricted but is preferably no greater than 2.0 mm and more preferably no greater than 1.5 mm. If the pitch is no greater than 2.0 mm, cotton in the first fiber layer composing the nonwoven fabric will be easily held in the second bottom sections of the second recesses, thus helping to further prevent splitting of the fiber layer by dissociation between the cotton and thermoplastic resin fibers in the first fiber layer, or interlayer separation between the fiber layers, at the second bottom sections. In addition, if the pitch is no greater than 2.0 mm, excreted fluid such as urine that has been supplied to the first surface side of the nonwoven fabric will be easily drawn in a spot-like manner into the second bottom sections of the second recesses disposed between adjacent protruded sections, thereby allowing even more excellent absorption performance to be exhibited as a top sheet (especially absorption rate and liquid migration), while moisture that has been released from the absorbent body will be even less prone to migrate from the second surface side of the nonwoven fabric to the first surface side. As a result, moisture that has been released from the absorbent body can be effectively confined in the spaces of the protruded sections, and the wearer can be even further protected from a feeling mustiness or the like.

Furthermore, when holes are provided in each of the pair of first perimeter wall sections of the second recesses as in the embodiment described above, the tensile force of the recesses with holes and the fibers in the adjacent protruded sections is released, allowing increased freedom of movement of the protruded sections as a whole or of the fibers forming the protruded sections, thereby increasing the flexibility of the protruded sections, and more specifically the flexibility of the protruded sections in the thickness direction of the top sheet, as well as the flexibility when the skin has slid in the lengthwise direction (first direction) or widthwise direction (and especially the widthwise direction) of the top sheet, and allowing a skin feel with a smooth touch sensation to be obtained. It is thus possible to impart, at the protruded sections, both an excellent hard/soft feeling (excellent softness in the thickness direction) and an excellent rough/smooth feeling in the lengthwise direction and widthwise direction (excellent smoothness on the front surface of the top sheet (particularly the widthwise direction)), to allow an excellent hard/soft feeling and rough/smooth feeling to be ensured for the top sheet as a whole, while also allowing a flexible feel on the skin to be achieved. In addition, if the first surface of the top sheet is smooth, it is possible to reduce physical irritation due to friction when it has rubbed against the skin of the wearer, and therefore eruption of the skin due to such physical irritation can also be made less likely to occur.

Furthermore, if holes are not provided in the second perimeter wall sections as in the embodiment described above, the level difference due to the second recesses will be less likely to catch on the skin when the skin has been rubbed in the lengthwise direction of the top sheet (i.e., in the direction in which the protruded sections and recesses extend), and therefore smoothness can be ensured in the lengthwise direction of the top sheet. That is, since such second perimeter wall sections in which holes are not formed are formed continuous with the first bottom sections and second bottom sections in an integral manner without seams, the skin is unlikely to sense the level difference of the first bottom sections due to the second recesses and will easily move along the protruded sections and recesses, when the skin slides in the lengthwise direction of the top sheet. This allows the flexibility of the protruded sections and the flexibility of the fibers to be exhibited to ensure smoothness in the lengthwise direction of the top sheet, and can make the skin even less likely to experience eruption caused by physical irritation.

Moreover, if holes are provided at locations of the first perimeter wall sections that are closer to the second bottom sections, as in the embodiment described above, then the holes will be present at locations further from the protruded sections and first bottom sections that are more likely to contact with the skin, helping to reduce the chance that the holes will contact with the skin of the wearer and making it less likely for the wearer to experience an uncomfortable feeling or sensation of foreign matter. This can more stably ensure smoothness when the skin is rubbed in the planar direction of the nonwoven fabric, and can more stably reduce the likelihood of eruption of the skin due to the aforementioned physical irritation.

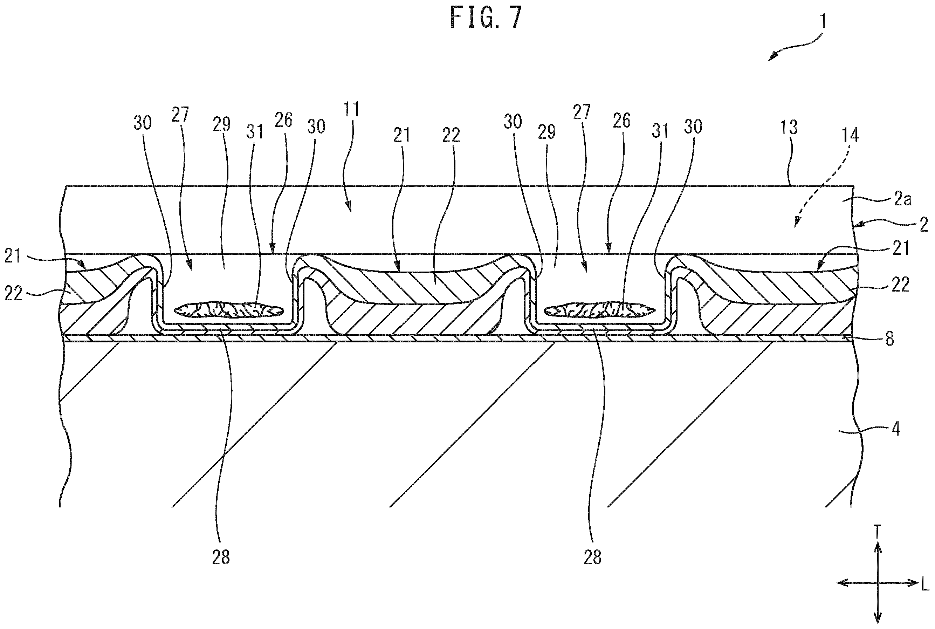

Incidentally, for the embodiment described above, as shown in Fig. 7, the holes 31 are formed by breaking the thermoplastic resin fibers contained in the top sheet 2 and not by melting of the thermoplastic resin fibers, and the perimeters of the holes 31 include, among the thermoplastic resin fibers in the nonwoven fabric composing the top sheet 2, the broken ends of broken fibers that have been broken by the holes 31. The broken ends of the broken fibers are ends formed by breaking, accomplished by pulling or physical cutting of the thermoplastic resin fibers in their lengthwise directions, and instead of being melted and rounded fiber ends with increased fiber sizes, as with ends formed by melting and breaking thermoplastic resin fibers, the fibers are in a tapered state formed by tearing, or else are in a state with virtually no change in fiber size. Thus, even when the skin of the wearer has been contacted with the perimeters of the holes 31, the absence of thermoplastic resin fibers hardened by melting helps to avoid any uncomfortable feeling due to stiffness or catching of the fibers, while also helping to prevent any feeling of the hardness or roughness of the top sheet 2.

Furthermore, as shown in Fig. 7, while some of the fibers among the thermoplastic resin fibers span across the interior spaces of the holes 31, some of the broken fibers have their broken ends protruding into the interior spaces of the holes 31. That is, the interior spaces of the holes 31 have a combination of thermoplastic resin fibers spanning across the interior spaces and thermoplastic resin fibers (the broken ends of the broken fibers) extending into them, so that the spaces are not completely open. Thus, even when the skin of the wearer has contacted with the holes 31, the thermoplastic resin fibers in the interior spaces reduce the level difference between the holes 31 and the first perimeter wall sections 29 or second bottom sections 28 of the second recesses 26, making it unlikely that the difference will be felt, and helping to avoid an uncomfortable feeling when the skin of the wearer has contacted them.

Incidentally, the open area percentage of the interior spaces of the holes (the proportion of the open area of the holes with respect to the area of the perimeter wall sections (the first perimeter wall sections) where the holes have been formed) is preferably in the range of 1 to 50%, more preferably in the range of 1.5 to 35% and even more preferably in the range of 2.5 to 20%. If the open area percentage is 1% or greater, it will be possible to impart adequate freedom to the protruded sections or the fibers in the protruded sections, and therefore to provide satisfactory flexibility to the protruded sections. If the open area percentage is 50% or lower, on the other hand, it will be easier to ensure the strength of the first perimeter wall sections in which the holes have been formed, and to make the borders of the hole perimeters less recognizable by touch. However, the open area percentage may be outside of this range, and set as desired, depending on the type of absorbent article and its purpose of use, etc.

Incidentally, for this embodiment, the top sheet 2 comprises holes 31 running through the second surfaces 2b, in each of the pair of first perimeter wall sections 29, 29 of the second recesses 26; however, according to the invention the holes do not have to be formed in the perimeter wall sections so long as it is possible to ensure the flexibility of the protruded sections.

In addition, since in the second bottom sections 28 of this embodiment, the nonwoven fabric composing the top sheet 2 is formed by being compacted in the direction from the first surfaces 2a to the second surfaces 2b, they have the highest fiber density of the top sheet 2, as mentioned above, as well as the highest rigidity. That is, in its thickness direction, the nonwoven fabric composing the top sheet 2 has a density gradient from the protruded sections with relatively low fiber density to the second bottom sections of the second recesses having the highest fiber density, and therefore excreted fluid such as urine supplied to the first surface side of the nonwoven fabric is easily drawn in a spot-like manner from the protruded sections to the second bottom sections of the second recesses, allowing excellent absorption performance (especially absorption rate and liquid migration properties) to be exhibited as a top sheet, and helping to prevent moisture that has been released from the absorbent body, from migrating from the second surface side to the first surface side of the nonwoven fabric. As a result, moisture that has been released from the absorbent body can be effectively confined in the spaces of the protruded sections, and the wearer can be even further protected from a feeling mustiness or the like.

In addition, the first surfaces 2a of the second bottom sections 28 (i.e. the inner side surfaces of the second recesses) and the second surfaces 2b (i.e. the absorbent body side surfaces) are formed in an essentially flat manner overall, and the second surfaces 2b of the second bottom sections 28 contact with the surface on the top sheet side of the absorbent body 4. When the second surfaces 2b of the second bottom sections 28 are thus formed essentially flat, it is possible to maximally increase the contact area between the second bottom sections 28 and the absorbent body 4, thereby making it possible to ensure that the second surfaces 2b of the second bottom sections 28 have joining regions that are as large as possible with respect to the surface of the top sheet side of the absorbent body 4. Thus, the top sheet 2 is less likely to detach from the absorbent body 4, and migration of excreted fluids between the top sheet 2 and the absorbent body 4 can proceed more smoothly. In particular, since the surface of the top sheet side of the absorbent body 4 is also formed essentially flat for this embodiment, the second surfaces 2b of the second bottom sections 28 are mutually joined with their surfaces in contact with the surface on the top sheet side of the absorbent body 4.

Therefore, the top sheet 2 can be stably and firmly joined with the absorbent body 4, thereby rendering them less likely to become detached from each other, and allowing migration of excreted fluids between the top sheet 2 and the absorbent body 4 to also be accomplished smoothly.

Incidentally, for this embodiment, the second surfaces 2b of the second bottom sections 28 are formed essentially flat; however, according to the invention the second surfaces of the second bottom sections do not necessarily have to be formed essentially flat so long as it is possible to adequately ensure the bonding strength or fluid mobility in the second bottom sections. Furthermore, while portions of the first bottom sections 22 in the embodiment described above are joined to the surface on the top sheet side of the absorbent body 4, according to the invention the first bottom sections do not necessarily need to be joined to the absorbent body so long as the top sheet and the absorbent body are stably joined. In this case as well, the second bottom sections are preferably joined to the absorbent body.

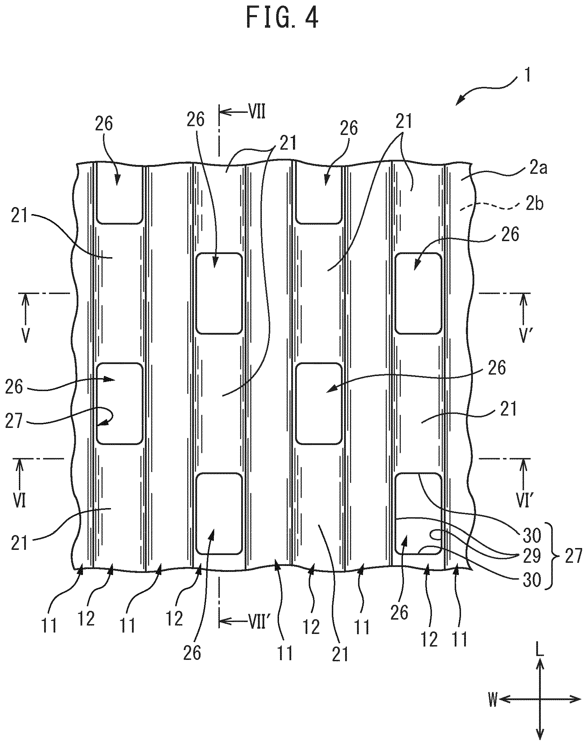

For the embodiment described above, the second recesses 26 are disposed on the top sheet 2 in a zigzag arrangement in the planar view, as shown in Fig. 4. Also, as shown in Fig. 5, the top sheet 2 has a construction in which at least two protruded sections 11 are situated between each second recess 26 and another second recess 26 adjacent to the second recess 26 in the widthwise direction. That is, the top sheet 2 has a protruded section 11, first recess 21 and protruded section 11 disposed in that order, between each pair of adjacent second recesses 26 in the widthwise direction.

If the top sheet 2 is constructed in this manner, it will be possible to more stably prevent detachment of the top sheet 2 from the absorbent body 4, and to help maintain the flexibility of the protruded sections. That is, if the top sheet 2 has the second recesses 26 joined to the absorbent body 4, this can potentially create tensile force in the widthwise direction, and since the first bottom sections 22 that have lower fiber density than the second bottom sections 28 and deform more easily than the second bottom sections 28, are disposed between adjacent second bottom sections 28, 28, the tensile force can be absorbed by deformation of the first bottom sections 22. This will help prevent interference with the freedom of the protruded sections 11 in the widthwise direction, therefore allowing the flexibility of the protruded sections 11 to be stably maintained.

Incidentally, for the embodiment described above, the top sheet 2 has a construction with at least two protruded sections 11 situated between each second recess 26 and the other second recesses 26 that are adjacent to that second recess 26 in the widthwise direction; however, according to the invention there is no limitation to such a construction, and the number of protruded sections between adjacent second recesses in the widthwise direction may be set as desired.

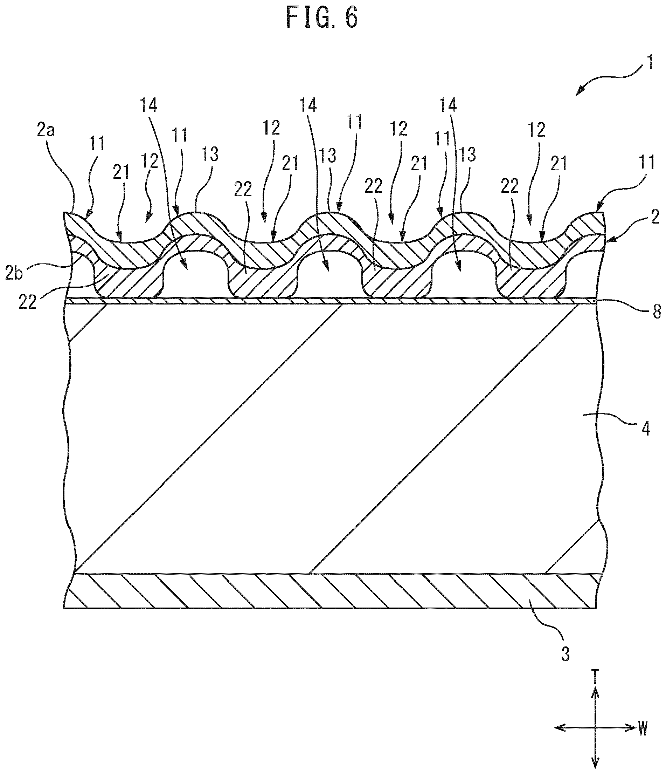

Furthermore, as shown in Fig. 4 and Fig. 6, in the top sheet 2, the construction in which first recesses 21 of the recesses 12 and the first recesses 21 of other recesses 12 adjacent to those recesses 12 are mutually adjacent has a portion that is continuous over the entire width in the widthwise direction. That is, the top sheet 2 has a portion where the first recesses 21, protruded sections 11 and first recesses 21 are arranged in a repeating manner in that order across the entire width in the widthwise direction, with the second recesses 26 being absent in that portion. This is because in the top sheet 2, the second recesses 26 are disposed in a zigzag fashion while the intervals between the second recesses 26 in the lengthwise direction are set wider than the sizes of the second recesses 26 in the lengthwise direction.

If the top sheet 2 has such a portion, it will be possible to utilize the ease of deformation of the first bottom sections 22 in the first recesses 21 to impart higher flexibility to the protruded sections 11. That is, if the top sheet 2 has a portion in which no second recesses 26 joined to the absorbent body 4 are present between adjacent protruded sections 11, then the first bottom sections 22, that have lower fiber density than the second bottom sections 28 of the second recesses 26 and deform more easily than the second bottom sections 28, can make the protruded sections 11 more easily follow movement of the wearer, and the protruded sections 11 can be fitted more stably onto the skin. Furthermore, since the flexibility of the first bottom sections 22 can be used to cause the protruded sections 11 to also deform more flexibly, it is possible to ensure even higher flexibility for the top sheet 2 as a whole.

Incidentally, in the top sheet 2 according to the embodiment described above, the construction in which the first recesses 21 of the recesses 12 and the first recesses 21 of other recesses 12 adjacent to those recesses 12 are mutually adjacent has a portion that is continuous over the entire width in the widthwise direction; however, according to the invention the top sheet does not need to have such a portion.

According to the invention, the protrusion-recess structure of the top sheet is not limited to the embodiment described above so long as it can exhibit the effect of the invention, and any protrusion-recess structure may be employed.

Moreover, the laminated construction of the nonwoven fabric used for the top sheet and the construction of the absorbent body are also not limited to the constructions of the embodiment described above so long as the effect of the invention can be exhibited, and any desired construction may be employed according to the desired properties and the purpose of use.

Another embodiment of the invention will now be explained. Incidentally, the aspects of the construction other than those differing from the embodiment described above (for example, the top sheet having a protrusion-recess structure and exhibiting weak acidity at least at the protruded sections of the protrusion-recess structure) are basically the same as the embodiment described above and will not be explained again here.

According to another embodiment of the invention, the nonwoven fabric used as the top sheet of the absorbent article is a fiber laminate consisting of three fiber layers. The fiber laminate (nonwoven fabric) is constructed with a second fiber layer forming the skin-facing surface (first surface) of the nonwoven fabric (skin-facing side fiber layer), a third fiber layer including thermoplastic resin fibers and including no cotton, which is adjacent to the non-skin-facing side surface of the second fiber layer (intermediate layer), and a first fiber layer including cotton, which is adjacent to the non-skin-facing side surface of the third fiber layer (non-skin-facing side fiber layer).

If the nonwoven fabric to be used as the top sheet is constructed in this manner, then the third fiber layer will be interposed as an intermediate layer between the second fiber layer forming the skin-facing surface of the top sheet and the first fiber layer that includes cotton, thereby separating the second fiber layer and the first fiber layer, such that excreted fluid such as urine that has been absorbed and held in the cotton of the first fiber layer that is situated on the non-skin-facing side will be even more unlikely to be transferred to the second fiber layer situated on the skin-facing side, and therefore to the skin of the wearer. Thus, the wearer is even less likely to perceive a wetted state or feel discomfort, and the skin of the wearer is also unlikely to become damp by excreted fluid (including moisture released from the absorbent body) (and as a result, skin eruption is less likely to be produced).

The following construction may be mentioned as a specific example of a three-layer fiber laminate according to another embodiment.

### [Skin-facing side fiber layer] (second fiber layer)

PET/PE core-sheath composite fibers with a fineness of 2.8 dtex, fiber lengths of 45 mm and a basis weight of 10 g/m², covered by a weak acidifying agent.

### [Intermediate layer] (third fiber layer)

PET/PE core-sheath composite fibers with a fineness of 2.8 dtex, fiber lengths of 45 mm and a basis weight of 10 g/m².

### [Non-skin-facing side fiber layer] (first fiber layer)

Blend of PET/PE core-sheath composite fibers with a fineness of 2.2 dtex, fiber lengths of 44 mm and a basis weight of 8 g/m², PET/PE core-sheath composite fibers with a fineness of 1.7 dtex, fiber lengths of 45 mm and a basis weight of 1 g/m², and cotton with a basis weight of 1 g/m².

According to yet another embodiment of the invention, the nonwoven fabric used as the top sheet of the absorbent article is a fiber laminate composed of three fiber layers, the fiber laminate (nonwoven fabric) being composed of a second fiber layer forming the skin-facing surface (first surface) of the nonwoven fabric (skin-facing side fiber layer), a first fiber layer including cotton, which is adjacent to the non-skin-facing side surface of the second fiber layer (intermediate layer), and a third fiber layer including thermoplastic resin fibers and including no cotton, which is adjacent to the non-skin-facing side surface of the first fiber layer (non-skin-facing side fiber layer).

If the nonwoven fabric used as the top sheet has such a construction, the structure will be such that the third fiber layer is interposed between the first fiber layer that contains cotton and the spaces on the second surface sides (non-skin-facing sides) of the protruded sections in the protrusion-recess structure of the nonwoven fabric, and it will thus be possible to ensure larger regions disposed on the non-skin-facing side of the third fiber layer, that confine moisture released from the absorbent body (i.e., regions formed by the spaces and the third fiber layer) (where the third fiber layer functions for auxiliary spaces to confine moisture released from the absorbent body). As a result, moisture released from the absorbent body can be effectively confined in the regions further toward the non-skin-facing side than the first fiber layer and the wearer is even less likely to feel mustiness, while the skin of the wearer is even less likely to become damp by excreted fluid (including moisture released from the absorbent body) (and as a result, skin eruption is even less likely to be produced).

The following construction may be mentioned as a specific example of a three-layer fiber laminate according to another embodiment.

### [Skin-facing side fiber layer] (second fiber layer)

PET/PE core-sheath composite fibers with a fineness of 2.8 dtex, fiber lengths of 45 mm and a basis weight of 10 g/m², covered by a weak acidifying agent.

### [Intermediate layer] (first fiber layer)

Blend of PET/PE core-sheath composite fibers with a fineness of 2.2 dtex, fiber lengths of 44 mm and a basis weight of 8 g/m², PET/PE core-sheath composite fibers with a fineness of 1.7 dtex, fiber lengths of 45 mm and a basis weight of 1 g/m², and cotton with a basis weight of 1 g/m².

### [Non-skin-facing side fiber layer] (third fiber layer)

PET/PE core-sheath composite fibers with a fineness of 2.8 dtex, fiber lengths of 45 mm and a basis weight of 10 g/m².

Incidentally, for each of the separate embodiments described above, the thermoplastic resin fibers in the third fiber layer are not particularly restricted and may be similar to the thermoplastic resin fibers in the first fiber layer or the second fiber layer. Also, for the separate embodiments, preferably each fiber layer has the same basis weight for the thermoplastic resin fibers, and the thermoplastic resin fibers contained in the second fiber layer and the third fiber layer have greater fiber sizes (finenesses) than the thermoplastic resin fibers in the first fiber layer. If the nonwoven fabric (fiber laminate) has such a construction, the total amount of thermoplastic resin fibers in the first fiber layer (the number of fibers) will necessarily be greater than in the second fiber layer and the third fiber layer, allowing the cotton-containing first fiber layer to have even greater strength. Furthermore, in such a nonwoven fabric, the thermoplastic resin fibers of the first fiber layer will tend to become entangled with the thermoplastic resin fibers of the second fiber layer or the third fiber layer, such that interlayer separation between each of the fiber layers will tend to be prevented even when the fiber layers composing the nonwoven fabric include cotton with relatively high rigidity.

According to yet another embodiment of the invention, the absorbent article has a back surface film on the non-skin-facing side of the back sheet (i.e., the side opposite the absorbent body), the back surface film having lower air permeability than the back sheet. If a back surface film having such low air permeability is present on the non-skin-facing side of the back sheet, then an air permeability gradient will be formed in which the air permeability is lower from the back sheet toward the back surface film, such that moisture released from the absorbent body toward the non-skin-facing side (i.e., the back sheet side) will tend to be drawn into the back surface film through the back sheet, and be eliminated to the exterior of the absorbent article. Thus, the amount of moisture released from the absorbent body to the skin-facing side (i.e., the opposite side from the back sheet) can be reduced, and therefore the wearer will be less likely to feel mustiness, and the skin of the wearer will be less likely to become damp by excreted fluid (including moisture released from the absorbent body) (resulting in even lower likelihood of skin eruption).

According to yet another embodiment of the invention, the absorbent body has compressed sections on the non-skin-facing side surface of the absorbent body. If the absorbent body has compressed sections on the non-skin-facing side surface, excreted fluid absorbed and held in the absorbent body will tend to pool in the compressed sections on the non-skin-facing side surface of the absorbent body, while being less likely to remain on the skin-facing side surface of the absorbent body, and therefore it will be possible to reduce the amount of moisture released from the absorbent body to the skin-facing side (i.e., the top sheet side), the wearer will be even less likely to feel mustiness, and the skin of the wearer will be even less likely to become damp by excreted fluid (resulting in skin eruption also being less likely to be produced).

According to yet another embodiment of the invention, the absorbent article has linear compressed sections running in the lengthwise direction and/or the widthwise direction of the absorbent article, on the non-skin-facing side surface of the absorbent body, and the absorbent body has a plurality of mutually separated punctiform compressed sections on the skin-facing side surface. If the absorbent body has such linear compressed sections on the non-skin-facing side surface, excreted fluid that has been absorbed and held in the absorbent body will easily pool on the non-skin-facing side surface of the absorbent body, in a state diffused along the linear compressed sections running in the lengthwise direction and/or the widthwise direction, while being unlikely to remain on the skin-facing side surface of the absorbent body, and it will therefore be possible to reduce the amount of moisture released from the absorbent body to the skin-facing side (i.e., the top sheet side), and to make it even less likely that the wearer will feel mustiness. In addition, if the absorbent body has the plurality of punctiform compressed sections on the skin-facing side surface, since the punctiform compressed sections have high fiber density and function as liquid-absorbing sections utilizing capillary movement, excreted fluid such as urine is easily absorbed in a spot-like manner into the absorbent body and is less likely to be released through the punctiform compressed sections to the skin-facing side (i.e., the top sheet side), with the result that the amount of moisture released from the absorbent body to the skin-facing side can be reduced and the wearer can be made even less likely to experience a feeling of mustiness. In addition, the skin of the wearer is even less likely to become damp by moisture, and as a result, skin eruption is even less likely to occur.

According to yet another embodiment of the invention, the top sheet has a center region running in the lengthwise direction and including the lengthwise axial line of the absorbent article (i.e., the center axis line in the widthwise direction running in the lengthwise direction), in the planar view, and a pair of outside regions located in both side sections adjacent to the center region in the widthwise direction, and running in the lengthwise direction, the absorbent article having joining sections on the second surface sides of the recesses of the top sheet, that join with the absorbent body, the joining sections extending in the lengthwise direction between the top sheet and the absorbent body and being disposed in a plurality of rows in the widthwise direction, and also being disposed so that the intervals between adjacent joining sections in the widthwise direction in the outside regions of the top sheet are larger than the intervals between adjacent joining sections in the widthwise direction in the center region of the top sheet. In the absorbent article having the joining sections joining the top sheet and absorbent body disposed in this manner, the pitch of the joining sections lying in the center region of the top sheet to which excreted fluid such as urine has been supplied is arranged so as to be denser than the joining sections in the outside regions, and it is therefore possible to firmly join the top sheet and the absorbent body at the center region that greatly contributes to absorption of excreted fluids, while also allowing the spaces of the protruded sections of the top sheet to be formed more compactly and in greater number than in the outside regions, thereby helping to more reliably ensure spaces for holding moisture released from the absorbent body to the skin-facing side (i.e. the top sheet side).

Incidentally, the absorbent article of the invention is not limited to the embodiments described above and can incorporate appropriate combinations, substitutions or modifications within a range that is not outside of the object and gist of the invention.

Moreover, the invention may be applied to any of various types of absorbent articles other than the tape-like disposable diaper according to the embodiment described above, such as a pants-type disposable diaper, sanitary napkin, panty liner or (light) incontinence pad. When the invention is to be applied in an absorbent article such as a sanitary napkin, it may be subjected to embossing, as joining means for integrating both the top sheet and the absorbent body. Such embossing allows firm joining to be achieved between the top sheet and the absorbent body, while also increasing the fiber density between the top sheet and the absorbent body, to help improve take up of excreted fluids such as menstrual blood from the top sheet to the absorbent body.

Incidentally, the ordinal terms "first" and "second" as used throughout the present description serve merely to distinguish between the numbered embodiments and are not used to mean any relative ordering, precedence or importance.

### REFERENCE SIGNS LIST

- 1: Disposable diaper (absorbent article)
- 2: Top sheet
- 201: First fiber layer
- 202: Second fiber layer
- 2a: First surface
- 2b: Second surface
- 3: Back sheet
- 4: Absorbent body
- 11: Protruded section
- 12: Recess
- 13: Top section
- 14: Space
- 21: First recess
- 22: First bottom section
- 26: Second recess
- 27: Perimeter wall section
- 28: Second bottom section
- 29: First perimeter wall section
- 30: Second perimeter wall section
- 31: Hole

## Claims

1. An absorbent article (1) including a liquid-permeable top sheet (2) situated on a side facing a skin of a wearer, a liquid-impermeable back sheet (3) situated on a side not facing the skin of the wearer, and an absorbent body (4) situated between both sheets,
wherein the top sheet (2) is a nonwoven fabric comprising at least two fiber layers including a first fiber layer (201) composed of cotton (203) and thermoplastic resin fibers (204) and a second fiber layer (202) composed of hydrophobic thermoplastic resin fibers (205),
the nonwoven fabric having a first surface (2a) which is a skin-facing side surface, formed by the second fiber layer (202), and a second surface (2b) which is a non-skin-facing side surface, facing the absorbent body (4), the nonwoven fabric being provided with a plurality of protruded sections (11) protruding toward the first surface (2a) and a plurality of recesses (12) depressed toward the second surface (2b), and formed between adjacent protruded sections,
the protruded sections (11) having spaces (14) where the second surface (2b) of the nonwoven fabric faces, and
the nonwoven fabric exhibiting weak acidity at least at the protruded sections.

2. The absorbent article according to claim 1, wherein the back sheet (3) has air permeability.

3. The absorbent article according to claim 1 or 2, wherein the first fiber layer (201) includes a cotton fiber mass.

4. The absorbent article according to any one of claims 1 to 3, wherein the absorbent article has a back surface film on the non-skin-facing side of the back sheet (3), the back surface film having lower air permeability than the back sheet.

5. The absorbent article according to any one of claims 1 to 4, wherein
the protruded sections (11) are laid out in a first direction on the first surface (2a) of the nonwoven fabric and provided at predetermined intervals in a second direction that is perpendicular to the first direction,
the recesses (12) are laid out in the first direction and provided between adjacent protruded sections (11) in the second direction, the recesses each having a first recess (21) comprising a first bottom section (22) situated more toward a second surface side than a location of a first surface side at top sections (13) of the protruded sections (11), and a plurality of second recesses (26) inside the first recess (21), provided in a discontinuous manner in the first direction, and depressed from the first bottom section toward the second surface, and
each of the second recesses (26) comprises perimeter wall section (27) extending from the first bottom section (22) in a direction of the second surface side, and second bottom section (28) having the highest fiber density of the nonwoven fabric, provided on edge of the second surface side of the perimeter wall section (27), so as to plug the edge.

6. The absorbent article according to claim 5, wherein a pitch of the second recesses (26) in the second direction is no greater than 2.0 mm.

7. The absorbent article according to any one of claims 1 to 6, wherein the protruded sections (11) are laid out in a first direction on the first surface (2a) of the nonwoven fabric and are provided at predetermined intervals in a second direction that is perpendicular to the first direction, while the protruded sections are laid out up to at least one of edges in the first direction of the absorbent body (4), or exceeding at least one of the edges.

8. The absorbent article according to any one of claims 1 to 7, wherein the absorbent body (4) has a compressed section on the non-skin-facing side surface.

9. The absorbent article according to claim 8, wherein
the absorbent article has a lengthwise direction (L), a widthwise direction (W) and a thickness direction (T) that are mutually perpendicular,
the compressed section is provided on the non-skin-facing side surface of the absorbent body (4), as a linear compressed section running in the lengthwise direction and/or the widthwise direction, and
the absorbent body has a plurality of mutually separated punctiform compressed sections on the skin-facing side surface.

10. The absorbent article according to any one of claims 1 to 9, wherein
the absorbent article (1) has a lengthwise direction (L), a widthwise direction (W) and a thickness direction (T) that are mutually perpendicular,
the top sheet (2) has a center region running in the lengthwise direction and including a lengthwise axial line of the absorbent article, in a planar view, and a pair of outside regions located in both side sections adjacent to the center region in the widthwise direction and running in the lengthwise direction,
the absorbent article has joining sections at sections of second surface sides of the recesses of the nonwoven fabric, that join with the absorbent body (4),
the joining sections extend in the lengthwise direction between the nonwoven fabric and the absorbent body and are disposed in a plurality of rows in the widthwise direction, and
the joining sections are disposed so that, among intervals between adjacent joining sections in the widthwise direction, intervals between joining sections lying in the outside regions of the top sheet are larger than intervals between joining sections lying in the center region in the widthwise direction of the top sheet.

## Patentansprüche

1. Absorbierender Artikel (1), der Folgendes einschließt: eine flüssigkeitsdurchlässige obere Lage (2), die sich auf einer Seite befindet, die einer Haut eines Trägers zugewandt ist, eine flüssigkeitsundurchlässige hintere Lage (3), die sich auf einer Seite befindet, die nicht der Haut des Trägers zugewandt ist, und einen Saugkörper (4), der sich zwischen beiden Lagen befindet,
wobei die obere Lage (2) ein Vliesstoff ist, der mindestens zwei Faserschichten umfasst, die Folgendes einschließen: eine erste Faserschicht (201), die aus Baumwolle (203) und thermoplastischen Harzfasern (204) besteht, und eine zweite Faserschicht (202), die aus hydrophoben thermoplastischen Harzfasern (205) besteht,
der Vliesstoff Folgendes aufweist: eine erste Oberfläche (2a), die eine Oberfläche auf der der Haut zugewandten Seite ist, die durch die zweite Faserschicht (202) ausgebildet ist, und eine zweite Oberfläche (2b), die eine Oberfläche auf der nicht der Haut zugewandten Seite ist, die dem Saugkörper (4) zugewandt ist, wobei der Vliesstoff mit Folgenden bereitgestellt ist: einer Vielzahl von vorstehenden Abschnitten (11), die in Richtung der ersten Oberfläche (2a) vorstehen, und einer Vielzahl von Vertiefungen (12), die in Richtung der zweiten Oberfläche (2b) abgesenkt sind und zwischen benachbarten vorstehenden Abschnitten ausgebildet sind,
die vorstehenden Abschnitte (11) Räume (14) aufweisen, wo die zweite Oberfläche (2b) des Vliesstoffs zugewandt ist, und
der Vliesstoff eine schwache Acidität zumindest an den vorstehenden Abschnitten besitzt.

2. Absorbierender Artikel nach Anspruch 1, wobei die hintere Lage (3) Luftdurchlässigkeit aufweist.

3. Absorbierender Artikel nach Anspruch 1 oder 2, wobei die erste Faserschicht (201) eine Baumwollfasermasse einschließt.

4. Absorbierender Artikel nach einem der Ansprüche 1 bis 3, wobei der absorbierende Artikel einen hinteren Oberflächenfilm auf der nicht der Haut zugewandten Seite der hinteren Lage (3) aufweist, wobei der hintere Oberflächenfilm eine geringere Luftdurchlässigkeit aufweist als die hintere Lage.

5. Absorbierender Artikel nach einem der Ansprüche 1 bis 4, wobei
die vorstehenden Abschnitte (11) in einer ersten Richtung auf der ersten Oberfläche (2a) des Vliesstoffs ausgelegt sind und in vorgegebenen Abständen in einer zweiten Richtung, das heißt senkrecht zu der ersten Richtung, bereitgestellt sind,
die Vertiefungen (12) in der ersten Richtung ausgelegt sind und zwischen benachbarten vorstehenden Abschnitten (11) in der zweiten Richtung bereitgestellt sind, wobei die Vertiefungen jeweils Folgendes aufweisen: eine erste Vertiefung (21), die einen ersten unteren Abschnitt (22) umfasst, der sich mehr in Richtung zu einer zweiten Oberflächenseite befindet als eine Stelle einer ersten Oberflächenseite an oberen Abschnitten (13) der vorstehenden Abschnitte (11), und eine Vielzahl von zweiten Vertiefungen (26) innerhalb der ersten Vertiefung (21), die in einer diskontinuierlichen Weise in der ersten Richtung bereitgestellt sind und von dem ersten unteren Abschnitt in Richtung zu der zweiten Oberfläche abgesenkt sind, und
jede der zweiten Vertiefungen (26) Folgendes umfasst: Umfangswandabschnitt (27), der sich von dem ersten unteren Abschnitt (22) in einer Richtung der zweiten Oberflächenseite erstreckt, und zweiten unteren Abschnitt (28), der die höchste Faserdichte des Vliesstoffs aufweist, der am Rand der zweiten Oberflächenseite des Umfangswandabschnitts (27) bereitgestellt ist, sodass der Rand ausgefüllt ist.

6. Absorbierender Artikel nach Anspruch 5, wobei ein Abstand der zweiten Vertiefungen (26) in der zweiten Richtung nicht größer als 2,0 mm ist.

7. Absorbierender Artikel nach einem der Ansprüche 1 bis 6, wobei die vorstehenden Abschnitte (11) in einer ersten Richtung der ersten Oberfläche (2a) des Vliesstoffs ausgelegt sind und in vorgegebenen Abständen in einer zweiten Richtung, die senkrecht zu der ersten Richtung ist, bereitgestellt sind, während die vorstehenden Abschnitte bis zu mindestens einem der Ränder in der ersten Richtung des Saugkörpers (4) ausgelegt sind oder mindestens einen der Ränder überschreiten.

8. Absorbierender Artikel nach einem der Ansprüche 1 bis 7, wobei der Saugkörper (4) einen komprimierten Abschnitt auf der Oberfläche der nicht der Haut zugewandten Seite aufweist.

9. Absorbierender Artikel nach Anspruch 8, wobei
der absorbierende Artikel eine Längsrichtung (L), eine Breitenrichtung (W) und eine Dickenrichtung (T) aufweist, die zueinander senkrecht liegen,
der komprimierte Abschnitt auf der Oberfläche der nicht der Haut zugewandten Seite des Saugkörpers (4) als linearer komprimierter Abschnitt, der in der Längsrichtung und/oder der Breitenrichtung verläuft, bereitgestellt ist und
der Saugkörper eine Vielzahl von voneinander getrennten punktförmigen komprimierten Abschnitten auf der Oberfläche der der Haut zugewandten Seite aufweist.

10. Absorbierender Artikel nach einem der Ansprüche 1 bis 9, wobei
der absorbierende Artikel (1) eine Längsrichtung (L), eine Breitenrichtung (W) und eine Dickenrichtung (T) aufweist, die zueinander senkrecht liegen,
die obere Lage (2) Folgendes aufweist: einen mittleren Bereich, der in der Längsrichtung verläuft und die axiale Längslinie des absorbierenden Artikels, in einer Draufsicht, einschließt, und ein Paar von äußeren Bereichen, die sich an beiden Seitenabschnitten benachbart zu dem mittleren Bereich in der Breitenrichtung befinden und in der Längsrichtung verlaufen,
der absorbierende Artikel Verbindungsabschnitte an Abschnitten der zweiten Oberflächenseiten der Vertiefungen des Vliesstoffs aufweist, die sich mit dem Saugkörper (4) verbinden,
die Verbindungsabschnitte sich in der Längsrichtung zwischen dem Vliesstoff und dem Saugkörper erstrecken und in einer Vielzahl von Reihen in der Breitenrichtung angeordnet sind und
die Verbindungsabschnitte derart angeordnet sind, dass unter den Abständen zwischen benachbarten Verbindungsabschnitten in der Breitenrichtung die Abstände zwischen Verbindungsabschnitten, die in den äußeren Bereichen der oberen Lage liegen, größer sind als die Abstände zwischen Verbindungsabschnitten, die in dem mittleren Bereich in der Breitenrichtung der oberen Lage liegen.

## Revendications

1. Article absorbant (1) comprenant une feuille de dessus perméable aux liquides (2) située sur un côté orienté vers la peau d'un utilisateur, une feuille de support imperméable aux liquides (3) située sur un côté non orienté vers la peau de l'utilisateur, et un corps absorbant (4) situé entre les deux feuilles,
dans lequel la feuille de dessus (2) est un tissu non tissé comportant au moins deux couches de fibres comprenant une première couche de fibres (201) composée de coton (203) et de fibres à base de résine thermoplastique (204) et une deuxième couche de fibres (202) composée de fibres à base de résine thermoplastique du type hydrophobe (205),
le tissu non tissé ayant une première surface (2a) qui est une surface du côté orienté vers la peau, formée par la deuxième couche de fibres (202), et une deuxième surface (2b) qui est une surface du côté non orienté vers la peau, orientée vers le corps absorbant (4), le tissu non tissé comportant une pluralité de sections saillantes (11) faisant saillie vers le première surface (2a) et une pluralité d'évidements (12) en retrait vers la deuxième surface (2b), et formés entre des sections saillantes adjacentes,
les sections saillantes (11) ayant des espaces (14) vers lesquels la deuxième surface (2b) du tissu non tissé est orientée, et
le tissu non tissé présentant une faible acidité au moins au niveau des sections saillantes.

2. Article absorbant selon la revendication 1, dans lequel la feuille de support (3) présente une perméabilité à l'air.

3. Article absorbant selon la revendication 1 ou la revendication 2, dans lequel la première couche de fibres (201) comprend une masse de fibres de coton.

4. Article absorbant selon l'une quelconque des revendications 1 à 3, dans lequel l'article absorbant a un film de surface de support sur le côté non orienté vers la peau de la feuille de support (3), le film de surface de support ayant une perméabilité à l'air inférieure par rapport à la feuille de support.

5. Article absorbant selon l'une quelconque des revendications 1 à 4, dans lequel
les sections saillantes (11) sont disposées dans une première direction sur la première surface (2a) du tissu non tissé et mises en œuvre selon des intervalles prédéterminés dans une deuxième direction qui est perpendiculaire par rapport à la première direction,
les évidements (12) sont disposés dans la première direction et mis en œuvre entre des sections saillantes adjacentes (11) dans la deuxième direction, les évidements ayant chacun un premier évidement (21) comportant une première section inférieure (22) située plus vers un deuxième côté de surface par rapport à un emplacement d'un premier côté de surface au niveau de sections supérieures (13) des sections saillantes (11), et une pluralité de deuxièmes évidements (26) à l'intérieur du premier évidement (21), mis en œuvre de manière discontinue dans la première direction, et en retrait par rapport à la première section inférieure vers la deuxième surface, et
chacun des deuxièmes évidements (26) comporte une section de paroi périmétrique (27) s'étendant depuis la première section inférieure (22) dans une direction du deuxième côté de surface, et une deuxième section inférieure (28) ayant la densité de fibres la plus élevée du tissu non tissé, mise en œuvre sur le bord du deuxième côté de surface de la section de paroi périmétrique (27), de manière à boucher le bord.

6. Article absorbant selon la revendication 5, dans lequel le pas des deuxièmes évidements (26) dans la deuxième direction n'est pas supérieur à 2,0 mm.

7. Article absorbant selon l'une quelconque des revendications 1 à 6, dans lequel les sections saillantes (11) sont disposées dans une première direction sur la première surface (2a) du tissu non tissé et sont mises en œuvre selon des intervalles prédéterminés dans une deuxième direction qui est perpendiculaire par rapport à la première direction, alors que les sections saillantes sont disposées jusqu'à au moins l'un des bords dans la première direction du corps absorbant (4), ou dépassant au moins l'un des bords.

8. Article absorbant selon l'une quelconque des revendications 1 à 7, dans lequel le corps absorbant (4) a une section comprimée sur la surface du côté non orienté vers la peau.

9. Article absorbant selon la revendication 8, dans lequel
l'article absorbant a une direction allant dans le sens de la longueur (L), une direction allant dans le sens de la largeur (W) et une direction allant dans le sens de l'épaisseur (T) qui sont mutuellement perpendiculaires,
la section comprimée est mise en œuvre sur la surface du côté non orienté vers la peau du corps absorbant (4), sous la forme d'une section comprimée linéaire qui va dans la direction allant dans le sens de la longueur et/ou dans la direction allant dans le sens de la largeur, et
le corps absorbant a une pluralité de sections comprimées ponctiformes mutuellement séparées sur la surface du côté orienté vers la peau.

10. Article absorbant selon l'une quelconque des revendications 1 à 9, dans lequel
l'article absorbant (1) a une direction allant dans le sens de la longueur (L), une direction allant dans le sens de la largeur (W) et une direction allant dans le sens de l'épaisseur (T) qui sont mutuellement perpendiculaires,
la feuille de dessus (2) a une région centrale qui va dans la direction allant dans le sens de la longueur et qui comprend une ligne axiale dans le sens de la longueur de l'article absorbant, dans une vue plane, et une paire de régions extérieures situées dans les deux sections latérales adjacentes par rapport à la région centrale dans la direction allant dans le sens de la largeur et qui vont dans la direction allant dans le sens de la longueur,
l'article absorbant a des sections d'assemblage au niveau de sections des deuxièmes côtés de surface des évidements du tissu non tissé. qui s'assemblent avec le corps absorbant (4),
les sections d'assemblage s'étendent dans la direction allant dans le sens de la longueur entre le tissu non tissé et le corps absorbant et sont disposées en une pluralité de rangées dans la direction allant dans le sens de la largeur, et
les sections d'assemblage sont disposées de telle sorte que, parmi des intervalles entre des sections d'assemblage adjacentes dans la direction allant dans le sens de la largeur, des intervalles entre des sections d'assemblage reposant dans les régions extérieures de la feuille de dessus sont supérieurs par rapport aux intervalles entre des sections d'assemblage reposant dans la région centrale dans la direction allant dans le sens de la largeur de la feuille de dessus.
